# EUROPEAN PATENT APPLICATION

(11) **EP 3 205 656 A1**
(43) Date of publication of application: **16.08.2017**
(21) Application number: 15849133.2
(22) Date of filing: 13.10.2015
(51) Int. Cl.: C07F 15/00, B01J 31/22, C07B 53/00, C07C 29/143, C07C 33/30, C07C 41/26, C07C 43/23, C07B 61/00, C07D 215/04, C07D 217/02, C07D 307/42

(54) **SOLID-SUPPORTED RUTHENIUM-DIAMINE COMPLEX, AND METHOD FOR MANUFACTURING OPTICALLY ACTIVE COMPOUND**

(30) Priority: 10.10.2014 JP 2014208681
(71) Applicant: Takasago International Corporation, Tokyo 144-8721 (JP)
(72) Inventor: TOUGE, Taichiro, Hiratsuka-shi Kanagawa 254-0073 (JP); NARA, Hideki, Hiratsuka-shi Kanagawa 254-0073 (JP)
(74) Representative: Zimmermann & Partner Patentanwälte mbB
(86) International application number: PCT/JP2015/078923
(87) International publication number: WO 2016/056669

(57) **Abstract**

Provided is a solid-supported ruthenium complex represented by general formula (1), (2) or (3). Further provided are: a method for manufacturing a reduction product by reducing an organic compound in the presence of the solid-supported ruthenium complex and a hydrogen donor; a method for manufacturing an optically active alcohol, characterized by reducing a carbonyl group in a carbonyl compound in the presence of the solid-supported ruthenium complex and a hydrogen donor; and a method for manufacturing an optically active amine, characterized by reducing an imino group of an imine compound in the presence of the solid-supported ruthenium complex and a hydrogen donor.

## Description

### Technical Field

The present invention relates to novel solid-supported ruthenium-diamine complexes, and to methods for selectively producing optically active alcohols and optically active amines, which are important as precursors for synthesis of pharmaceuticals and functional materials, by using the complexes as catalysts.

### Background Art

Many asymmetric reactions including asymmetric reductions have been developed, and many asymmetric reactions have been reported which use, as catalysts for these reactions, asymmetric metal complexes having optically active phosphine ligands. Meanwhile, many reports have been presented which recite that complexes in which optically active nitrogen compounds are coordinated to transition metals such as ruthenium, rhodium, or iridium have excellent performances as catalysts for asymmetric synthesis reactions. In this connection, many optically active nitrogen compounds of various types have been developed so far to enhance the performances of such catalysts (see Chem Rev. (1992) p. 1051; J. Am. Chem. Soc. 117 (1995) p. 7562; J. Am. Chem. Soc. 118 (1996) p. 2521; J. Am. Chem. Soc. 118 (1996) p. 4916, etc.).

Among others, M. Wills et al. have reported complexes in which a diamine moiety and an aromatic compound (arene) moiety coordinated to the ruthenium complex are linked through a carbon chain, and these complexes are known to exhibit higher activities than conventional catalysts (J. Am. Chem. Soc. 127 (2005) p. 7318; J. Org. Chem. 71 (2006) p. 7035; Org. Biomol. Chem. 5 (2007) p. 1093; Org. Lett. 9 (2007) p. 4659; J. Organometallic Chem. 693 (2008) p. 3527; Dalton Trans. 39 (2010) p. 1395, etc.).

In addition, the present inventor and others have reported complexes in which a diamine moiety and an aromatic compound (arene) moiety coordinated to the ruthenium complex are linked through an oxygen atom-containing side chain, and these complexes are known to exhibit much higher activities (see Japanese Patent Application Publication No. 2012-67071, J. Am. Chem. Soc. 133 (2011) p. 14960, etc.).

Meanwhile, several catalysts have been developed in which conventionally used complexes each having a diamine moiety and an aromatic compound (arene) moiety coordinated to the ruthenium complex which are not linked through a carbon chain or an oxygen atom-containing side chain, such as those reported in J. Am. Chem. Soc. 117 (1995) p. 7562; J. Am. Chem. Soc. 118 (1996) p. 2521; and J. Am. Chem. Soc. 118 (1996) p. 4916 mentioned above, are supported on solids such as silica gel (see Org. Lett. 6 (2004) p. 169; Chem. Commun. (2004) p. 2070; Eur. J. Org. Chem. (2005) p. 3221, etc.).

### Summary of Invention

Methods using these solid-supported complexes are very advantageous in, for example, that the catalysts can be reused, and the dissolved metal remaining after reaction can be reduced. However, conventional complexes result in insufficient catalytic activity, insufficient enantiomeric excess, or the like in some cases depending on the target reaction or the reaction substrate, and hence there is a demand for development of a complex which has a still higher activity and a still higher selectivity, and which undergoes less metal dissolution into the reaction liquid after reaction. An object of the present invention is to solve these problems.

To solve the above-described problems, the present inventors and others have found that supporting, on a solid support, a complex having a chain-like moiety which links an aromatic compound moiety (including an arene moiety) (hereinafter, "aromatic compound moiety" include "arene moiety" in this description) coordinated to a ruthenium complex having an optically active diamine to the diamine moiety makes it possible to obtain a novel solid-supported ruthenium-diamine complex which has a high catalytic activity, achieves a good enantiomeric excess, and is suitable for industrial use because of its simple production method. This finding has led to the completion of the present invention.

Specifically, the present invention includes the following contents. A solid-supported ruthenium complex represented by general formula (1): 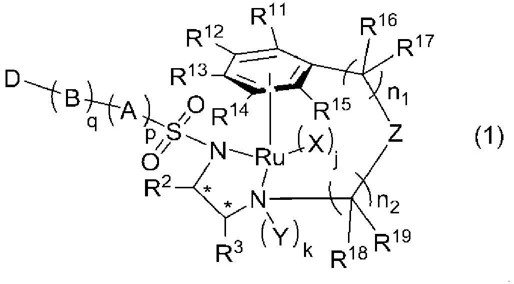 wherein
each * represents an asymmetric carbon atom;
R² and R³ each independently represent an alkyl group having 1 to 10 carbon atoms; a phenyl group optionally having a substituent(s) selected from alkyl groups having 1 to 10 carbon atoms, alkoxy groups having 1 to 10 carbon atoms, and halogen atoms; or a cycloalkyl group having 3 to 8 carbon atoms and optionally having a substituent(s) selected from alkyl groups having 1 to 10 carbon atoms, provided that R² and R³, taken together, may form a ring;
R¹¹, R¹², R¹³, R¹⁴, and R¹⁵ each independently represent a hydrogen atom, an alkyl group having 1 to 10 carbon atoms, or an alkoxy group having 1 to 10 carbon atoms;
R¹⁶, R¹⁷, R¹⁸, and R¹⁹ each independently represent a hydrogen atom, a hydroxyl group, an alkyl group having 1 to 10 carbon atoms, or an alkoxy group having 1 to 10 carbon atoms, provided that R¹⁶ and R¹⁷, together with the carbon atom to which R¹⁶ and R¹⁷ are bonded, may form a carbonyl group, and provided that R¹⁸ and R¹⁹, together with the carbon atom to which R¹⁸ and R¹⁹ are bonded, may form a carbonyl group;
Z represents an oxygen atom or methylene (-CH₂-);
n₁ represents 1 or 2, and n₂ represents an integer of 1 to 3;
Y represents a hydrogen atom;
X represents a trifluoromethanesulfonyloxy group, a p-toluenesulfonyloxy group, a methanesulfonyloxy group, a benzenesulfonyloxy group, a hydrogen atom, or a halogen atom;
j and k each independently represent 0 or 1, provided that j+k is not 1;
A represents a divalent group selected from optionally substituted linear or branched alkylene groups having 1 to 20 carbon atoms, optionally substituted cycloalkylene groups having 3 to 20 carbon atoms, optionally substituted arylene groups having 6 to 20 carbon atoms, optionally substituted heterocyclic groups, an oxygen atom, a sulfur atom, 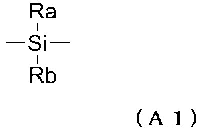 wherein Ra and Rb each independently represent a hydrogen atom, an alkyl group having 1 to 10 carbon atoms, or an aryl group having 6 to 10 carbon atoms, and 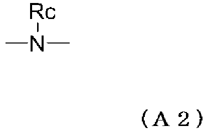 wherein Rc represents a hydrogen atom, an alkyl group having 1 to 10 carbon atoms, or an aryl group having 6 to 10 carbon atoms,
   or a group formed by bonding two or more of these divalent groups, provided that either side of each of formula (A1) and formula (A2) may be bonded to B;
B represents 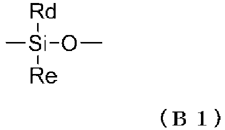 wherein Rd and Re each independently represent a hydrogen atom, an alkyl group having 1 to 10 carbon atoms, an aryl group having 6 to 10 carbon atoms, or an alkoxy group having 1 to 10 carbon atoms, 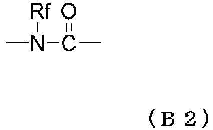 wherien Rf represents a hydrogen atom or an alkyl group having 1 to 10 carbon atoms, 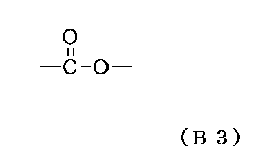 an oxygen atom, or a sulfur atom, provided that either side of each of formula (B1), formula (B2), and formula (B3) may be bonded to D;
D represents a solid support; and
p and q each independently represent 0 or 1.

A solid-supported ruthenium complex represented by general formula (2): 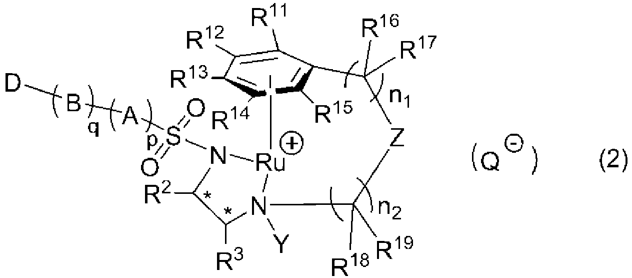 wherein
each * represents an asymmetric carbon atom;
R², R³, R¹¹, R¹², R¹³_{,} R¹⁴, R¹⁵, R¹⁶, R¹⁷, R¹⁸, R¹⁹ ,Z, n₁, n₂, Y A, B, D, p, and q are as defined above; and
Q⁻ represents a counter anion.

A solid-supported ruthenium complex represented by general formula (3): 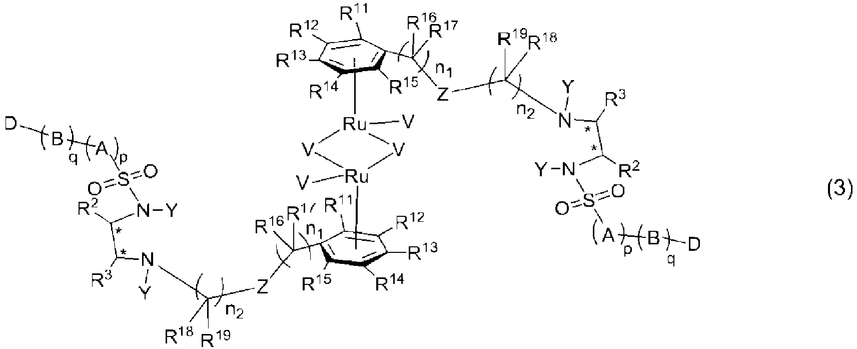 wherein
each * represents an asymmetric carbon atom;
R², R³, R¹¹_{,} R¹²_{,} R¹³, R¹⁴, R¹⁵, R¹⁶, R¹⁷, R¹⁸, R¹⁹, Z, n₁, n₂, Y, A, B, D, p, and q are as defined above; and
each V represents a halogen atom.

A method for producing a reduction product, comprising reducing an organic compound in the presence of the solid-supported ruthenium complex represented by any one of general formulae (1) to (3) and a hydrogen donor.

A method for producing an optically active alcohol, comprising reducing a carbonyl group of a carbonyl compound in the presence of the solid-supported ruthenium complex represented by any one of general formulae (1) to (3) and a hydrogen donor.

A method for producing an optically active amine, comprising reducing an imino group of an imine compound in the presence of the solid-supported ruthenium complex represented by any one of general formulae (1) to (3) and a hydrogen donor.

Any one of the above-described production methods, wherein the hydrogen donor is selected from formic acid, alkali metal formates, and alcohols having a hydrogen atom at an α-position carbon atom of a hydroxyl-bearing carbon.

Any one of the above-described production methods, wherein the hydrogen donor is hydrogen.

A reduction catalyst, comprising the solid-supported ruthenium complex represented by any one of general formulae (1) to (3).

The above-described reduction catalyst, which is an asymmetric reduction catalyst.

The present invention provides novel complexes in each of which a ruthenium-diamine complex having a chain-like moiety linking an aromatic compound moiety coordinated to ruthenium and a diamine moiety to each other is supported on a solid support. Each of the solid-supported ruthenium-diamine complexes of the present invention has a very high catalytic activity and can be used for reduction of carbonyl groups, imino groups, or the like. Hence, the solid-supported ruthenium-diamine complex of the present invention is useful as a catalyst for various types of hydrogenation. In addition, a complex of the present invention having a ligand in an optically active form is excellent in stereoselectivity and gives a high enantiomeric excess. Moreover, because of the insolubility, the solid-supported ruthenium-diamine complexes of the present invention can easily be separated and recovered from the reaction systems, and exhibit higher catalytic activities than conventional complexes even in the reuse after recovery, and hardly undergo decrease in catalytic activity even after repetition of reuse. Furthermore, the catalysts can be not only recovered and reused, but also removed by filtration after the reaction, so that the amount of metal remaining in the solution after the reaction, that is, in the product can be greatly reduced. Hence, the catalysts are useful and industrially excellent. The use of the solid-supported ruthenium-diamine complexes of the present invention makes it possible to selectively produce optically active alcohols and optically active amines, which are useful as raw materials for producing pharmaceuticals and functional materials and the like.

### Description of Embodiments

The solid-supported ruthenium complexes of the present invention represented by general formulae (1) to (3) each have an aromatic compound moiety coordinated to a ruthenium atom and a diamine moiety in which one of the nitrogen atoms is linked to the aromatic compound moiety through a chain-like moiety and the other nitrogen atom has a sulfonyl group from which a side chain having a terminal bonded to a solid support extends.

In addition, the solid-supported ruthenium complexes represented by general formulae (1) and (2) each have a tridentate ligand in which the two nitrogen atoms of the diamine ligand are covalently or coordinately bonded to the ruthenium atom, and the aromatic compound moiety linked to the diamine is also coordinately bonded to the ruthenium atom. In addition, the solid-supported ruthenium complexes represented by general formulae (1) and (2) each have the chain-like moiety which links the aromatic compound moiety and the diamine moiety to each other, with a terminal of a side chain extending from the sulfonyl group bonded to the diamine moiety being bonded to the solid support.

Each symbol * in general formulae (1), (2), and (3) indicates that the carbon atom marked with the symbol * may be an asymmetric carbon atom in some cases. When the carbon atom is an asymmetric carbon atom, the carbon atom may be an optically active form thereof, a mixture of optically active forms, or a racemic form (including a racemic compound). In a preferred embodiment of the present invention, when these carbon atoms are asymmetric carbon atoms, these carbon atoms are in an optically active form.

The solid-supported ruthenium complex represented by general formula (2) is the same as the solid-supported ruthenium complex represented by general formula (1), except that the bond Ru-X is replaced with an ionic bond of Ru⁺-Q⁻.

The solid-supported ruthenium complex represented by general formula (3) is a complex (dimer) dimerized through halogen atoms V, in which the aromatic compound moieties are coordinated to the ruthenium atoms. The solid-supported ruthenium complex represented by general formula (3) is not only useful as an intermediate for producing the solid-supported ruthenium complex represented by general formula (1) or (2), but also active as a reduction catalyst by itself.

The complexes represented by general formulae (1) to (3) of the present invention are described.

### <Solid-Supported Ruthenium Complex of General Formula (1)>

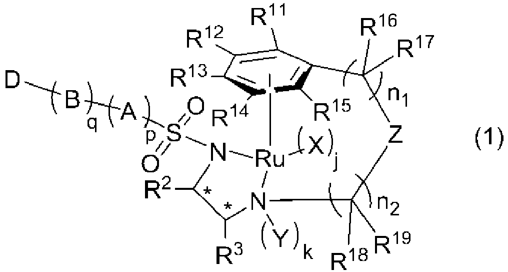

wherein
each * represents an asymmetric carbon atom.

In general formula (1) of the present invention, R² and R³ each independently represent an alkyl group having 1 to 10 carbon atoms; a phenyl group optionally having a substituent(s) selected from alkyl groups having 1 to 10 carbon atoms, alkoxy groups having 1 to 10 carbon atoms, and halogen atoms; or a cycloalkyl group having 3 to 8 carbon atoms and optionally having a substituent(s) selected from alkyl groups having 1 to 10 carbon atoms. R² and R³, taken together, may form a ring. In addition, it is preferable that both R² and R³ be a phenyl group optionally having a substituent(s) selected from alkyl groups having 1 to 10 carbon atoms, alkoxy groups having 1 to 10 carbon atoms, and halogen atoms, and it is more preferable that both R² and R³ be an unsubstituted phenyl group.

The alkyl group having 1 to 10 carbon atoms represented by each of R² and R³ in general formula (1) of the present invention may be a linear or branched alkyl group having 1 to 10 carbon atoms, and preferably 1 to 5 carbon atoms. Specific examples of the alkyl group include a methyl group, an ethyl group, a n-propyl group, an isopropyl group, a n-butyl group, an isobutyl group, a sec-butyl group, a tert-butyl group, a n-pentyl group, a n-hexyl group, a n-heptyl group, a n-octyl group, a n-nonyl group, a n-decyl group, and the like. The alkyl group is preferably a methyl group, an ethyl group, a n-propyl group, an isopropyl group, a n-butyl group, an isobutyl group, a sec-butyl group, a tert-butyl group, or a n-pentyl group.

Next, the alkyl groups having 1 to 10 carbon atoms, the alkoxy groups having 1 to 10 carbon atoms, and the halogen atoms which may be present as the substituents on the phenyl group represented by each of R² and R³ in general formula (1) of the present invention are described. Note that the phenyl group can have one or multiple substituents.

The alkyl groups having 1 to 10 carbon atoms serving as the substituents can be selected from, for example, the above-described groups defined as the alkyl group having 1 to 10 carbon atoms represented by each of R² and R³ in formula (1).

The alkoxy groups having 1 to 10 carbon atoms serving as the substituents include linear or branched alkoxy groups having 1 to 10 carbon atoms, and preferably 1 to 5 carbon atoms. Specific examples of the alkoxy groups include methoxy groups, ethoxy groups, n-propoxy groups, isopropoxy groups, n-butoxy groups, isobutoxy groups, sec-butoxy groups, tert-butoxy groups, n-pentyloxy groups, n-hexyloxy groups, n-heptyloxy groups, n-octyloxy groups, n-nonyloxy groups, n-decyloxy group, and the like. The alkoxy groups are each preferably a methoxy group, an ethoxy group, a n-propoxy group, an isopropoxy group, a n-butoxy group, an isobutoxy group, a sec-butoxy group, a tert-butoxy group, or a n-pentyloxy group.

Examples of the halogen atoms serving as the substituents include fluorine atoms, chlorine atoms, and bromine atoms.

The cycloalkyl group having 3 to 8 carbon atoms represented by each of R² and R³ in general formula (1) of the present invention may be a monocyclic, fused-cyclic, or bridged cycloalkyl group having 3 to 8 carbon atoms, and preferably 5 to 8 carbon atoms. Specific examples thereof include a cyclopropyl group, a cyclobutyl group, a cyclopentyl group, a cyclohexyl group, a cycloheptyl group, a cyclooctyl group, and the like.

The alkyl groups having 1 to 10 carbon atoms serving as the substituents which may be present on the cycloalkyl group represented by each of R² and R³ in formula (1) can be selected from, for example, the above-described groups defined as the alkyl group having 1 to 10 carbon atoms represented by each of R² and R³ in formula (1), and are each preferably a methyl group, an isopropyl group, a tert-butyl group, or the like. The cycloalkyl group can have one or multiple substituents.

In addition, when R² and R³, taken together, form a ring, R² and R³ forms a 4 to 8-membered, preferably, 5 to 8-membered cycloalkane ring together with the adjacent carbon atom. Preferred cycloalkane rings include a cyclopentane ring, a cyclohexane ring, and a cycloheptane ring, and these rings may have substituents selected from alkyl groups having 1 to 10 carbon atoms. Specifically, the alkyl groups serving as the substituents include methyl groups, isopropyl groups, tert-butyl groups, and the like. The ring can have one or multiple substituents.

R¹¹, R¹², R¹³, R¹⁴, and R¹⁵ in the aromatic compound moiety represented by general formula (1) of the present invention each independently represent a hydrogen atom, an alkyl group having 1 to 10 carbon atoms, or an alkoxy group having 1 to 10 carbon atoms. Moreover, R¹¹, R¹², R¹³, R¹⁴, and R¹⁵ are preferably a hydrogen atom or an alkyl group having 1 to 5 carbon atoms, and R¹¹, R¹², R¹³, R¹⁴, and R¹⁵ are more preferably any one of a hydrogen atom, a methyl group, and an ethyl group.

The alkyl group having 1 to 10 carbon atoms represented by each of R¹¹, R¹², R¹³, R¹⁴, and R¹⁵ can be selected from the above-described groups defined as the alkyl group having 1 to 10 carbon atoms represented by each of R² and R³.

The alkoxy group having 1 to 10 carbon atoms represented by each of R¹¹, R¹², R¹³, R¹⁴, and R¹⁵ may be a linear or branched alkoxy group having 1 to 10 carbon atoms, and preferably 1 to 5 carbon atoms. Specific examples of the alkoxy group include a methoxy group, an ethoxy group, a n-propoxy group, an isopropoxy group, a n-butoxy group, an isobutoxy group, a sec-butoxy group, a tert-butoxy group, a n-pentyloxy group, a n-hexyloxy group, a n-heptyloxy group, a n-octyloxy group, a n-nonyloxy group, a n-decyloxy group, and the like. The alkoxy group is preferably a methoxy group, an ethoxy group, a n-propoxy group, an isopropoxy group, a n-butoxy group, an isobutoxy group, a sec-butoxy group, a tert-butoxy group, or a n-pentyloxy group.

R¹⁶, R¹⁷, R¹⁸, and R¹⁹ which represent substituents on the carbon atoms in the chain-like moiety linking the aromatic compound moiety and the diamine moiety to each other in general formula (1) each independently represent a hydrogen atom, a hydroxyl group, an alkyl group having 1 to 10 carbon atoms, or an alkoxy group having 1 to 10 carbon atoms. In addition, R¹⁶, R¹⁷, R¹⁸, and R¹⁹ are more preferably a hydrogen atom.

In addition, n₁ in general formula (1) represents 1 or 2, and n₂ represents an integer of 1 to 3. n₁ is preferably 1, and n₂ is preferably 2.

The alkyl group having 1 to 10 carbon atoms represented by each of R¹⁶, R¹⁷, R¹⁸, and R¹⁹ can be selected from the above-described groups defined as the alkyl group having 1 to 10 carbon atoms represented by each of R² and R³.

The alkoxy group having 1 to 10 carbon atoms represented by each of R¹⁶, R¹⁷, R¹⁸, and R¹⁹ can be selected from the above-described groups defined as the alkoxy group having 1 to 10 carbon atoms represented by each of R¹¹ to R¹⁵.

R¹⁶ and R¹⁷, together with the carbon atom to which R¹⁶ and R¹⁷ are bonded, may form a carbonyl group.

R¹⁸ and R¹⁹, together with the carbon atom to which R¹⁸ and R¹⁹ are bonded, may form a carbonyl group.

Preferred examples of the -(-C(R¹⁶)(R¹⁷)-)n₁- group include a -CH₂-group, a -CH₂-CH₂-group, a -CH(CH₃)-group, a -CO-group, and the like, but are not limited to these groups.

Preferred examples of the -(-C(R¹⁸)(R¹⁹)-)n₂-group include a -CH₂-group, a -CH₂-CH₂-group, a -CH(CH₃)-group, a -CO-group, and the like, but are not limited to these groups.

In general formula (1), Z is an oxygen atom or methylene (-CH₂-).

In general formula (1), j and k are each independently 0 or 1, provided that j+k is not 1. In other words, when k is 1, j is also 1, while when k is 0, j is also 0.

Y represents a hydrogen atom.

In general formula (1), X represents a trifluoromethanesulfonyloxy group, a p-toluenesulfonyloxy group, a methanesulfonyloxy group, a benzenesulfonyloxy group, a hydrogen atom, or a halogen atom. X is preferably a hydrogen atom or a halogen atom, specifically, such as a fluorine atom, a chlorine atom, or a bromine atom, and X is particularly preferably a chlorine atom, for example.

The hydrogen atom represented by each of Y in general formula (1) and X in general formula (1) may be not only an ordinary hydrogen atom, but also an isotope of hydrogen atom. A preferred isotope may be a deuterium atom.

A in general formula (1) of the present invention represents a divalent group selected from optionally substituted linear or branched alkylene groups having 1 to 20 carbon atoms, optionally substituted cycloalkylene groups having 3 to 20 carbon atoms, optionally substituted arylene groups having 6 to 20 carbon atoms, optionally substituted heterocyclic groups, an oxygen atom, a sulfur atom, formula (A1) and formula (A2), or a group formed by bonding two or more of these divalent groups.

The optionally substituted linear or branched alkylene group having 1 to 20 carbon atoms represented by A may be one obtainable by removing one hydrogen atom from a linear or branched alkyl group having 1 to 20 carbon atoms, and is preferably an alkylene group having 1 to 10 carbon atoms. Specifically, the alkylene group may be a methylene group, an ethylene group, a trimethylene group, a tetramethylene group, a n-propylene group, a n-butylene group, a n-hexylene group, a n-heptylene group, a n-octylene group, a n-decylene group, or the like.

Substituents which may be present on the alkylene group include halogen atoms such as fluorine atoms, chlorine atoms, bromine atoms, and iodine atoms, the above-described alkoxy groups, carboxyl groups, oxo groups (=O), hydroxyl groups, amino groups, cyano groups, nitro groups, and the like. The alkylene group can have one or multiple substituents.

The optionally substituted cycloalkylene group having 3 to 20 carbon atoms represented by A may be a monocyclic, fused-cyclic, or bridged cycloalkylene group, and specifically may be a divalent group obtainable by removing two hydrogen atoms from a cyclopropane ring, a cyclobutane ring, a cyclopentane ring, a cyclohexane ring, or a cycloheptane ring. A cycloalkylene group having 3 to 10 carbon atoms is preferable.

Substituents which may be present on the cycloalkylene group include halogen atoms such as fluorine atoms, chlorine atoms, bromine atoms, and iodine atoms, alkyl groups having 1 to 10 carbon atoms, the above-described alkoxy groups, carboxyl groups, oxo groups (=O), hydroxyl groups, amino groups, cyano groups, nitro groups, and the like. The cycloalkylene group can have one or multiple substituents.

The optionally substituted arylene group having 6 to 20 carbon atoms represented by A may be a monocyclic or fused-cyclic arylene group, and specifically may be a divalent group obtainable by removing two hydrogen atoms from benzene, naphthalene, indene, anthracene, azulene, pyrene, tetralin, or the like. Arylene groups having 6 to 15 carbon atoms are preferable.

Substituents which may be present on the arylene group include halogen atoms such as fluorine atoms, chlorine atoms, bromine atoms, and iodine atoms, alkyl groups having 1 to 10 carbon atoms, the above-described alkoxy groups, carboxyl groups, hydroxyl groups, amino groups, cyano groups, nitro groups, and the like. The arylene group can have one or multiple substituents.

The optionally substituted heterocyclic group represented by A is preferably a heterocyclic group having 2 to 14 carbon atoms and containing at least one heteroatom, and more preferably a heterocyclic group having 2 to 10 carbon atoms and containing at least one heteroatom, and particularly preferably a heterocyclic group having 2 to 6 carbon atoms and containing at least one heteroatom. Examples of the heterocyclic group having 2 to 14 carbon atoms and containing at least one heteroatom include aliphatic heterocyclic groups having 2 to 14 carbon atoms and containing at least one heteroatom and aromatic heterocyclic groups having 2 to 14 carbon atoms and containing at least one heteroatom. Specific heterocyclic groups include divalent groups obtainable by removing two hydrogen atoms from pyridine, furan, thiophene, pyrrole, pyrazole, imidazole, oxazole, thiazole, triazole, pyrimidine, indole, pyrazine, quinoline, isoquinoline, quinoxaline, coumarin, benzofuran, benzothiophene, or the like.

Substituents which may be present on the heterocyclic group include halogen atoms such as fluorine atoms, chlorine atoms, bromine atoms, and iodine atoms, alkyl groups having 1 to 10 carbon atoms, the above-described alkoxy groups, carboxyl groups, hydroxyl groups, amino groups, cyano groups, nitro groups, and the like. The heterocyclic group can have one or multiple substituents.

Formula (A1) represented by A is described.

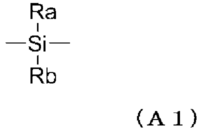

In formula (A1), Ra and Rb each independently represent a hydrogen atom, an alkyl group having 1 to 10 carbon atoms, or an aryl group having 6 to 10 carbon atoms.

The alkyl group having 1 to 10 carbon atoms represented by Ra and Rb can be selected from the above-described groups defined as the alkyl group having 1 to 10 carbon atoms represented by each of R² and R³.

The aryl group having 6 to 10 carbon atoms represented by each of Ra and Rb may be an aromatic monocyclic group, an aromatic polycyclic group, or an aromatic fused-cyclic group, and is preferably an aromatic monocyclic group. In addition, the aryl group may have, as the substituents, one or multiple substituents selected from alkyl groups having 1 to 10 carbon atoms and alkoxy groups having 1 to 6 carbon atoms. The aryl group specifically may be a phenyl group, a tolyl group, a xylyl group, or the like.

Formula (A2) represented by A is described.

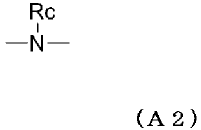

In formula (A2), Rc represents a hydrogen atom, an alkyl group having 1 to 10 carbon atoms, or an aryl group having 6 to 10 carbon atoms.

The alkyl group having 1 to 10 carbon atoms represented by Rc can be selected from the above-described groups defined as the alkyl group having 1 to 10 carbon atoms represented by each of R² and R³.

The aryl group having 6 to 10 carbon atoms represented by Rc can be selected from the above-described groups defined as the aryl group having 6 to 10 carbon atoms represented by each of Ra and Rb.

Note that either side of each of formula (A1) and formula (A2) may be bonded to B.

Specific examples of the skeleton of A include -alkylene-, -arylene-, -cycloalkylene-, -alkylene-cycloalkylene-, -arylene-cycloalkylene-, -arylene-alkylene-, -alkylene-arylene-alkylene-, -alkylene-O-, -alkylene-O-alkylene-, -alkylene-O-arylene-, -alkylene-S-, -alkylene-S-alkylene-, -alkylene-S-arylene-, -formula (A1)-alkylene-, -formula (A1)-arylene-, -formula (A2)-alkylene-, -formula (A2)-arylene-, -alkylene-heterocycle-, -arylene-heterocycle-, -O-heterocycle-, -S-heterocycle-, -formula (A1)-heterocycle-, -formula (A2)-heterocycle-, and the like.

Preferred skeletons of A include -arylene-alkylene-, -arylene-, and -arylene-alkylene-formula (A2)-alkylene-arylene-. The skeleton of A is more preferably phenylene-alkylene-, -phenylene-, or -phenylene-alkylene-formula (A2)-alkylene-phenylene-, and is more preferably -phenylene-alkylene having 1 to 3 carbon atoms-, -phenylene-, or -phenylene-alkylene having 1 to 3 carbon atoms-formula (A2) in which Rc is hydrogen - oxo-substituted alkylene having 1 to 3 carbon atoms-phenylene-.

B in general formula (1) of the present invention represents formula (B1), formula (B2), or formula (B3) described below, an oxygen atom, or a sulfur atom.

Formula (B1) represented by B is described.

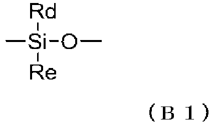

In formula (B1), Rd and Re each independently represent a hydrogen atom, an alkyl group having 1 to 10 carbon atoms, an aryl group having 6 to 10 carbon atoms, or an alkoxy group having 1 to 10 carbon atoms.

The alkyl group having 1 to 10 carbon atoms represented by each of Rd and Re can be selected from the above-described groups defined as the alkyl group having 1 to 10 carbon atoms represented by each of R² and R³.

The aryl group having 6 to 10 carbon atoms represented by each of Rd and Re may be an aromatic monocyclic group, an aromatic polycyclic group, or an aromatic fused-cyclic group, and is preferably an aromatic monocyclic group. In addition, the aryl group may have one or multiple substituents selected from alkyl groups having 1 to 10 carbon atoms and alkoxy groups having 1 to 6 carbon atoms (the alkyl groups having 1 to 10 carbon atoms and serving as the substituents can be selected from the groups defined as the alkyl group having 1 to 10 carbon atoms represented by each of R² and R³, and the alkoxy groups having 1 to 6 carbon atoms and serving as the substituents can be selected from the groups defined as the alkoxy group having 1 to 10 carbon atoms represented by each of R¹¹ to R¹⁵). The aryl group represented by each of Rd and Re specifically may be a phenyl group, a tolyl group, a xylyl group, or the like. The alkoxy group having 1 to 10 carbon atoms represented by each of Rd and Re can be selected from the above-described groups defined as the alkoxy group having 1 to 10 carbon atoms represented by each of R¹¹ to R¹⁵.

Formula (B2) represented by B is described.

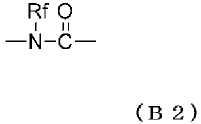

In formula (B2), Rf represents a hydrogen atom or an alkyl group having 1 to 10 carbon atoms.

The alkyl group having 1 to 10 carbon atoms represented by Rf can be selected from the above-described groups defined as the alkyl group having 1 to 10 carbon atoms represented by R² and R³.

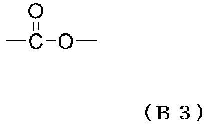

Note that either side of each of formula (B1), formula (B2), and formula (B3) may be bonded to D.

In addition, p and q in general formula (1) each independently represent 0 or 1, and p and q are more preferably, for example, such that p is 1 and q is 1, or p is 1 and q is 0.

The solid support represented by D in general formula (1) refers to any support which is solid at normal temperature and hardly soluble in various organic solvents and water, and examples thereof include silica gel, polystyrene, and the like. The silica gel may be acidic silica gel, neutral silica gel, basic silica gel such as NH₂ silica gel, or amorphous silica, or mesoporous silica typified by MCM-41, SBA-15, and FSM-16, or the like. The polystyrene includes various types of polystyrene obtained by polymerization of styrene with any equivalents of a copolymer of divinylbenzene or the like.

In addition, other solid supports include JandaJels^{™} and TantaGel (registered trademark), which are polystyrene-like supports having increased swelling properties, and the like. Still other solid supports include trityl resins, PEG resins, Halolink^{™} Resin, oxime resins, peptides, as well as inorganic supports such as iron oxide, and the like.

### <Solid-Supported Ruthenium Complex of General Formula (2)>

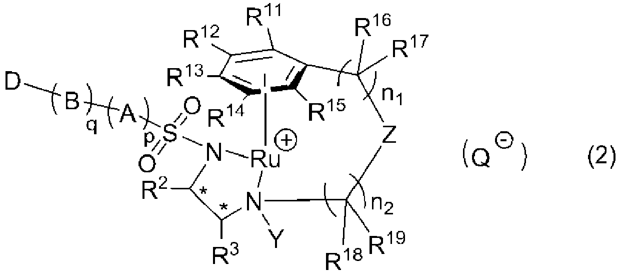

wherein each * represents an asymmetric carbon atom.

In formula (2), R², R³, R¹¹, R¹², R¹³, R¹⁴, R¹⁵, R¹⁶, R¹⁷, R¹⁸, R¹⁹ Z, n₁, n₂, Y, A, B, D, p, and q are as defined above.

In formula (2), Q⁻ represents a counter anion. Specific counter anions include alkylsulfonyloxy ions or arenesulfonyloxy ions such as a trifluoromethanesulfonyloxy ion (TfO⁻), a p-toluenesulfonyloxy ion (TsO⁻), a methanesulfonyloxy ion (MsO⁻), a benzenesulfonyloxy ion (BsO⁻); and ions such as BF4⁻, SbF₆⁻ , CF₃COO⁻, CH₃COO⁻, PF6⁻, NO₃⁻, ClO₄⁻, SCN⁻, OCN⁻, ReO₄⁻ , MoO₄⁻, BPh₄⁻, B(C₆F₅)₄⁻, and B(3,5-(CF₃)₂C₆F₃)₄⁻.

### <Solid-Supported Ruthenium Complex of General Formula (3) >

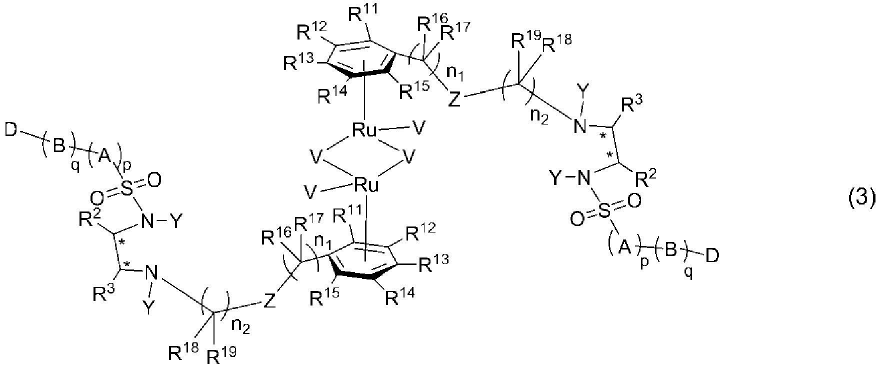

wherein each * represents an asymmetric carbon atom.

In formula (3) R², R³, R¹¹, R¹², R¹³, R¹⁴, R¹⁵, R¹⁶, R¹⁷, R¹⁸, R¹⁹, Z, n₁, n₂, Y, A, B, D, p, and q are as defined above.

In formula (3), each V represents a halogen atom.

The halogen atom represented by each V in general formula (3) is any one of chlorine, bromine, or iodine atoms. All Vs in general formula (3) may be the same halogen atoms or a combination of different halogen atoms.

### [Methods for Producing Complexes]

In the following schemes, *, R², R³, X, Y, Z, V, Q⁻, R¹¹, R¹², R¹³, R¹⁴, R¹⁶, R¹⁶, R¹⁷, R¹⁸, R¹⁹, A, B, D, n₁, n₂, p, q, j, and k are as defined above.

### <Methods for Producing Complex of General Formula (3)>

A diamine compound having a cyclohexadiene and represented by general formula (5), which is an intermediate for producing the complex of general formula (3), can be synthesized by a method of scheme 1 or 2.

As shown in scheme 1, the compound of general formula (5) can be synthesized by reacting a diamine compound represented by general formula (6) in which a sulfonyl group has been introduced on one side thereof with a cyclohexadiene derivative represented by general formula (7) according to the description in Supporting Information (see the right column on Page 14962) of J. Am. Chem. Soc. 133 (2011) p. 14960.

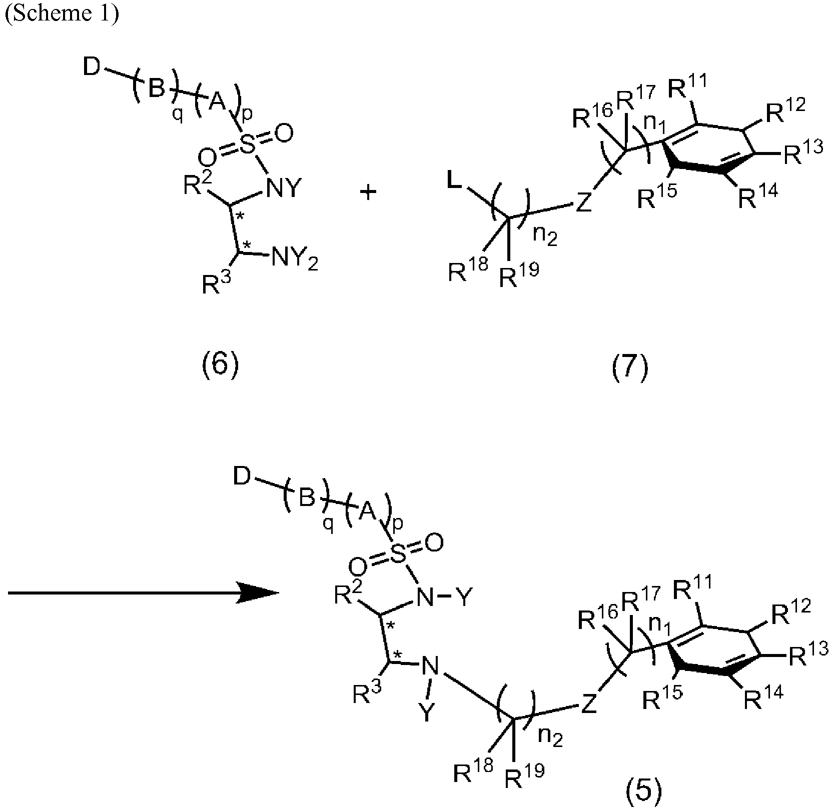

In scheme 1, L represents a leaving group.

As shown in scheme 2, general formula (5) can also be synthesized by sulfonylation of a compound represented by general formula (9) with a sulfonyl chloride represented by general formula (8) or the like.

Note that the compound represented by general formula (9) can be synthesized by reacting the compound of general formula (7) in scheme 1 shown above with a diamine.

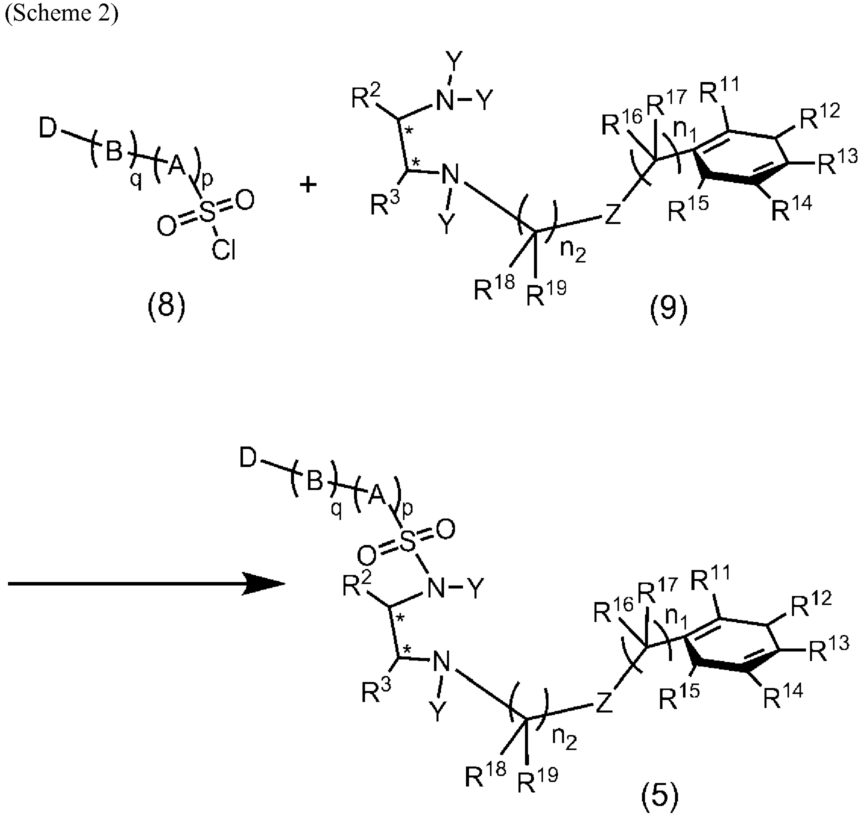

The solid-supported ruthenium complex represented by general formula (3) can be produced by reacting the diamine compound represented by general formula (5) with ruthenium metal such as a ruthenium halide. The production method is shown in scheme 3.

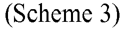

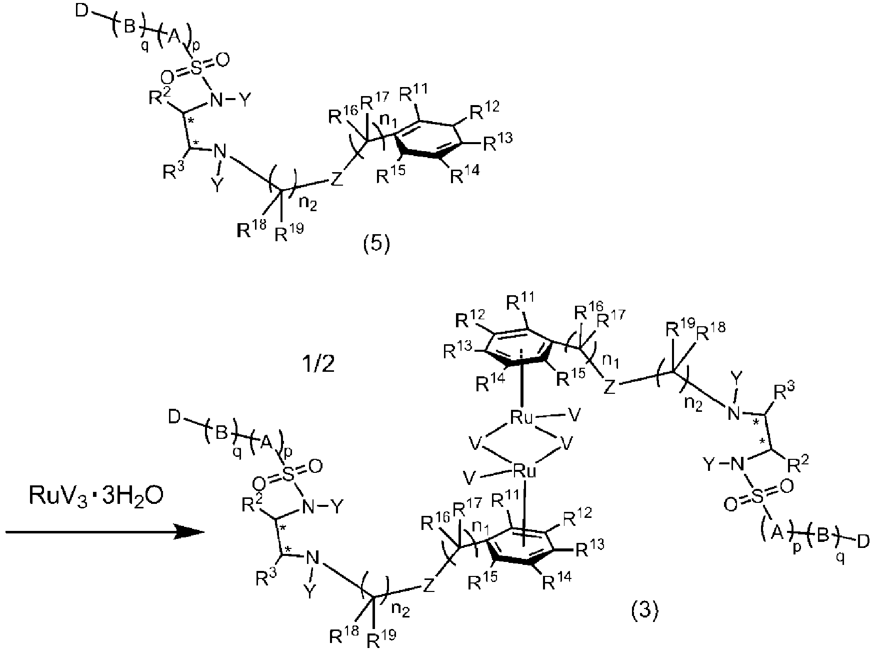

Regarding a method for synthesizing general formula (3) from the compound of general formula (5), general formula (3) can be produced according to the method described in Org. Lett. 9 (2007), p. 4659.

In scheme 3, the halogenoruthenium represented by RuV₃ is not particularly limited, and, for example, it is possible to use ruthenium chloride, ruthenium bromide, ruthenium iodide, or a hydrate thereof, and it is preferable to use ruthenium chloride or a hydrate thereof.

Solvents used include, but are not particularly limited to, aliphatic alcohols such as 2-propanol, n-butanol, 2-butanol, n-pentanol, 2-pentanol, 3-pentanol, 3-methyl-1-butanol, cyclopentanol, 3-methoxy-1-propanol, 2-methoxyethanol, 2-ethoxyethanol, 2-isopropoxyethanol, n-hexanol, 3-methoxy-1-butanol, 3-methoxy-3-methyl-1-butanol, 2-hexanol, 3-hexanol, cyclohexanol, n-heptanol, 2-heptanol, 3-heptanol, cycloheptanol, n-octanol, 2-octanol, 3-octanol, 4-octanol, and cyclooctanol; aromatic alcohols such as phenol, benzyl alcohol, 1-phenylethanol, 2-phenylethanol, o-cresol, m-cresol, p-cresol, 2-methylbenzyl alcohol, 3-methylbenzyl alcohol, and 4-methylbenzyl alcohol; and diols and derivatives thereof such as ethylene glycol, 1,2-propanediol, 1,3-propanediol, 1,2-butanediol, 1,3-butanediol, 1,4-butanediol, ethylene glycol-n-butyl ether, ethylene glycol-iso-butyl ether, and ethylene glycol-n-hexyl ether. One of these solvents may be used alone, or two or more thereof may be used as a mixture. Combining two or more solvents makes it possible to adjust the boiling point of the solvent within a desired range and to adjust the reaction temperature in a case where the reaction is carried out under reflux. For example, a mixture of an alcohol with a small amount of water may be used.

The amount of the compound of general formula (5) used is 1 to 20 mole equivalents, preferably 1 to 10 mole equivalents, and more preferably 1 to 5 mole equivalents to the ruthenium atoms.

The amount of the solvent used is not particularly limited, as long as the halogenoruthenium or the hydrate thereof can be dissolved at reaction temperature. For example, the volume of the solvent used is 2 to 100 times the halogenoruthenium or the hydrate thereof (i.e., the volume of the solvent is 2 to 100 mL relative to 1 g of the halogenoruthenium or the hydrate thereof; hereinafter, the same shall apply), and preferably 2 to 75 times the halogenoruthenium or the hydrate thereof.

The reaction temperature varies depending on the solvent used, and is 60°C or above and preferably 100°C or above, while 200°C or below and preferably 160°C or below from the viewpoint of reaction efficiency.

The reaction time varies depending on the reaction substrate used, and is 30 minutes to 20 hours, and preferably 1 hour to 12 hours. This production method is preferably conducted in an inert gas such as nitrogen gas or argon gas.

### <Method for Producing Complex of General Formula (1)>

### [Method for Producing Complex of General Formula (1) in which j=1 and X is Halogen Atom]

As shown in scheme 4A, a complex of general formula (1) in which j=1 and X is a halogen atom can be synthesized by treating the complex of general formula (3) with a base in the presence of a suitable solvent.

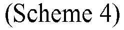

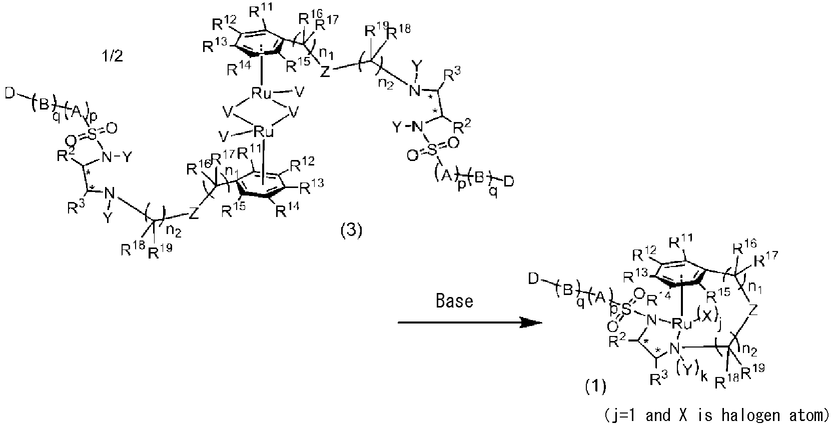

Solvents used include, but are not particularly limited to, halogenated solvents such as methylene chloride, dichloroethane, chloroform, and trifluoroethanol; aromatic hydrocarbons such as toluene and xylene; ethers such as diisopropyl ether and tetrahydrofuran; and alcohols such as methanol, ethanol, 2-propanol, n-butanol, 2-butanol, and n-pentanol. Of these solvents, chloroform, dichloromethane, and isopropanol are particularly preferable. One of these solvents may be used alone, or two or more thereof may be used as a mixture. Combining two or more solvents makes it possible to adjust the boiling point of the solvent within a desired range and to adjust the reaction temperature in a case where the reaction is carried out under reflux. For example, a mixture of an alcohol with a small amount of water may be used.

Bases used include inorganic bases such as sodium hydrogen carbonate, sodium carbonate, potassium hydrogen carbonate, potassium carbonate, lithium hydroxide, lithium hydrogen carbonate, lithium carbonate, cesium carbonate, magnesium hydroxide, magnesium carbonate, calcium hydroxide, and calcium carbonate; and amines such as triethylamine, tripropylamine, tributylamine, pyridine, and triisopropylamine. Of these bases, triethylamine is particularly preferable. The amount of the base used is 0.2 to 20 mole equivalents, and preferably 1.0 to 10 mole equivalents to ruthenium atoms.

The amount of the solvent used is not particularly limited, but is, for example, 2 times by volume to 50 times by volume relative to the substrate (solvent mL/substrate g).

The reaction temperature is not particularly limited, and is 0°C to a temperature not higher than the boiling point of the solvent, and is preferably room temperature to 80°C.

The reaction time varies depending on the reaction substrate used, and is 30 minutes to 20 hours, and preferably 1 hour to 12 hours. This production method is preferably conducted in an inert gas such as nitrogen gas or argon gas.

### [Method for Producing Complex of General Formula (1) in which j=0]

As shown in scheme 5, a complex of general formula (1) in which j=0 can be synthesized by treating the complex of general formula (3) with a base in the presence of a suitable solvent.

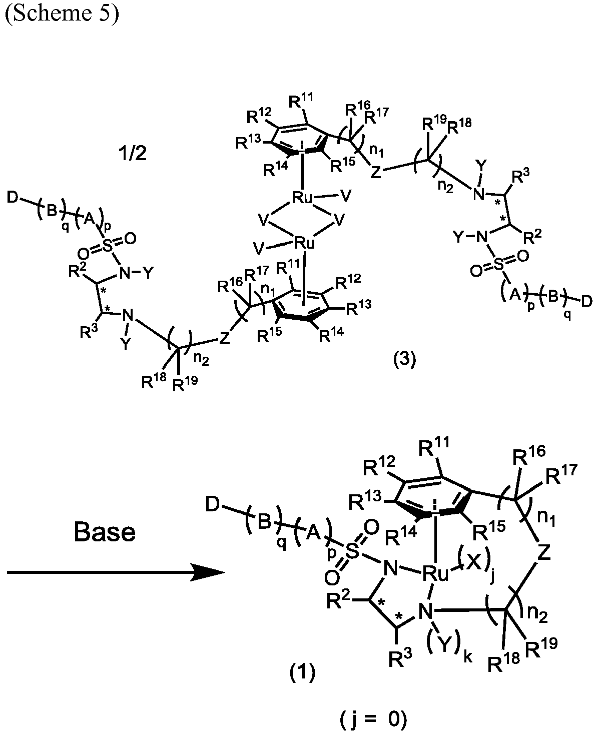

Solvents used include, but are not particularly limited to, halogenated solvents such as methylene chloride, dichloroethane, chloroform, and trifluoroethanol; aromatic hydrocarbons such as toluene and xylene; and ethers such as diisopropyl ether and tetrahydrofuran. Of these solvents, dichloromethane, toluene, and tetrahydrofuran are particularly preferable. One of these solvents may be used alone, or two or more thereof may be used as a mixture. Combining two or more solvents makes it possible to adjust the boiling point of the solvent within a desired range and to adjust the reaction temperature in a case where the reaction is carried out under reflux.

Bases used include inorganic bases such as sodium hydroxide, sodium hydrogen carbonate, sodium carbonate, potassium hydroxide, potassium hydrogen carbonate, potassium carbonate, lithium hydroxide, lithium hydrogen carbonate, lithium carbonate, cesium carbonate, magnesium hydroxide, magnesium carbonate, calcium hydroxide, and calcium carbonate. Of these bases, sodium hydroxide and potassium hydroxide are particularly preferable. The amount of the base used is 1 to 20 mole equivalents, and preferably 1.0 to 10 mole equivalents to ruthenium atoms.

The amount of the solvent used is not particularly limited, and is, for example, 2 times by volume to 20 times by volume relative to the substrate (solvent mL/substrate g).

The reaction temperature is not particularly limited, and is 0°C to a temperature not higher than the boiling point of the solvent, and is preferably room temperature to 80°C.

The reaction time varies depending on the reaction substrate used, and is 30 minutes to 20 hours, and preferably 1 hour to 12 hours. This production method is preferably conducted in an inert gas such as nitrogen gas or argon gas.

### [Method for Producing Complex of General Formula (1) in which j=1 and X is Trifluoromethanesulfonyloxy Group, p-Toluenesulfonyloxy Group, Methanesulfonyloxy Group, or Benzenesulfonyloxy Group]

A complex of general formula (1) in which j=1 and X is a trifluoromethanesulfonyloxy group, a p-toluenesulfonyloxy group, a methanesulfonyloxy group, or a benzenesulfonyloxy group can be synthesized by treating the complex of general formula (1) in which j=0 obtained by scheme 5 with a corresponding suitable acid.

The production method is shown in scheme 6.

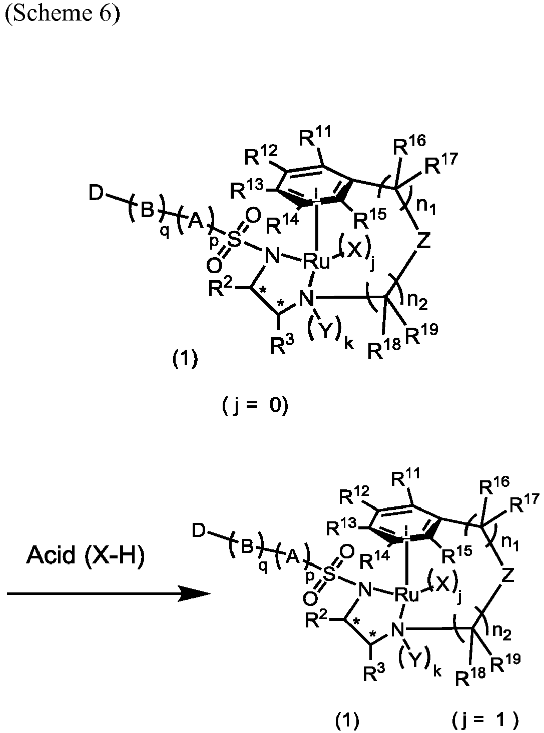

In scheme 6, X is a trifluoromethanesulfonyloxy group, a p-toluenesulfonyloxy group, a methanesulfonyloxy group, or a benzenesulfonyloxy group.

The acids (X-H) used include trifluoromethanesulfonic acid, p-toluenesulfonic acid, methanesulfonic acid, benzenesulfonic acid, and the like. The amount of the acid used is 1 to 20 mole equivalents, and preferably 1.0 to 10 mole equivalents to ruthenium.

Examples of the solvent used include, but are not particularly limited to, halogenated solvents such as methylene chloride, dichloroethane, chloroform, and trifluoroethanol; aromatic hydrocarbons such as toluene and xylene; ethers such as diisopropyl ether and tetrahydrofuran; and alcohols such as methanol and ethanol.

The amount of the solvent used is not particularly limited, and is, for example, 2 times by volume to 20 times by volume relative to the substrate (solvent mL/substrate g).

The reaction temperature is not particularly limited, and is 0°C to a temperature not higher than the boiling point of the solvent, and preferably room temperature to 80°C.

The reaction time varies depending on the reaction substrate used, and is 30 minutes to 20 hours, and preferably 1 hour to 12 hours. This production method is preferably conducted in an inert gas such as nitrogen gas or argon gas.

### [Methods for Producing Complex of General Formula (1) in which j=1 and X is Hydrogen Atom]

As shown in scheme 7, a complex of general formula (1) in which j=1 and X is a hydrogen atom can be synthesized by reacting the complex represented by general formula (3) with formic acid, hydrogen gas, or the like.

The production method is shown in scheme 7.

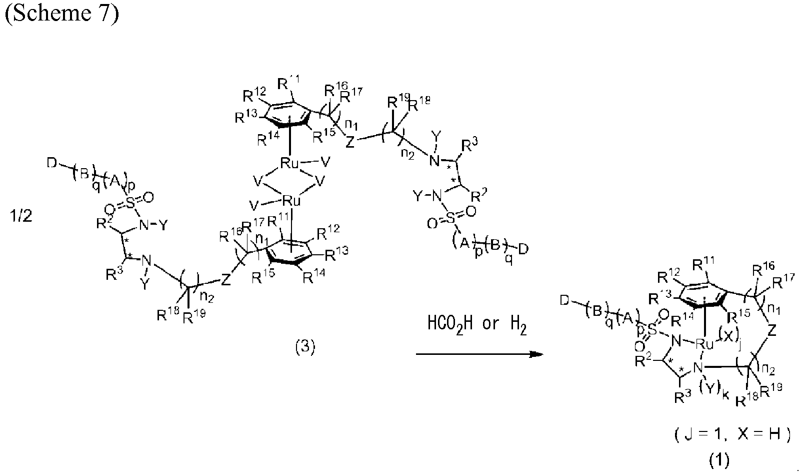

The formic acid or hydrogen gas used is equimolar or more relative to ruthenium atoms.

Solvents used are not particularly limited, and include alcohols such as methanol, ethanol, and isopropanol, aromatic hydrocarbons such as toluene and xylene, halogenated hydrocarbons such as dichloromethane and 1,2-dichloroethane, aprotic polar solvents such as acetonitrile and N,N-dimethylformamide, and ethers such as diethyl ether and tetrahydrofuran, and the like, of which methanol and dichloromethane are preferable. A single solvent may be used, or multiple solvents may be used as a mixture.

The amount of the solvent used is not particularly limited, and is, for example, 2 times by volume to 20 times by volume relative to the substrate (solvent mL/substrate g).

The reaction temperature is not particularly limited, and is 0°C to a temperature not higher than the boiling point of the solvent, and preferably room temperature to 80°C.

The reaction time varies depending on the reaction substrate used, and is 30 minutes to 20 hours, and preferably 1 hour to 12 hours. This production method is preferably conducted in an inert gas such as nitrogen gas or argon gas.

Alternatively, the complex of general formula (1) in which j=1 and X is a hydrogen atom can also be synthesized by (a) reacting the complex of general formula (1) in which j=0 obtained by scheme 5 with formic acid, hydrogen gas, or the like, (b) reacting the complex of general formula (1) in which j=1 and X is a trifluoromethanesulfonyloxy group, a p-toluenesulfonyloxy group, a methanesulfonyloxy group, or a benzenesulfonyloxy group obtained by scheme 6 with formic acid, hydrogen gas, or the like, or (c) reacting a complex of general formula (2) synthesized by scheme 8 described later with formic acid, hydrogen gas, or the like.

The reaction conditions of these reactions are the same as those of scheme 7.

### <Method for Producing Complex of General Formula (2)>

The solid-supported ruthenium complex represented by general formula (2) can be produced by reacting a complex of general formula (1) in which j=1 and X is a halogen atom with a metal salt represented by G-Q.

The production method is shown in scheme 8.

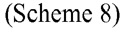

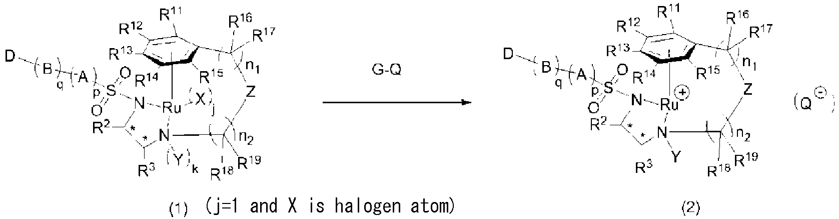

Examples of the metal G in G-Q include silver (Ag), sodium (Na), potassium (K), lithium (Li), and the like. Q may be an alkanesulfonyloxy or arenesulfonyloxy such as trifluoromethanesulfonyloxy (TfO), p-toluenesulfonyloxy (TsO), methanesulfonyloxy (MsO), or benzenesulfonyloxy (BsO), and further may be BF₄, SbF₆, CF₃COO, CH₃COO, PF₆, NO₃, ClO₄, SCN, OCN, ReO₄, MoO₄ BPh₄, B(C₆F₅)₄, B(3,5-(CF₃)₂C₆F₃)₄, or the like.

Examples of the metal salt represented by G-Q include AgOTf, AgOTs, AgOMs, AgOBs, AgBF₄, AgSbF₆, CF₃COOAg, CH₃COOAg, AgPF₆, AgNO₃, AgClO₄, AgSCN, AgOCN, AgReO₄, AgMoO₄, NaOTf, NaBF₄, NaSbF₆, CF₃COONa, CH₃COONa, NaPF₆, NaNO₃, NaClO₄, NaSCN, KOTf, KBF₄, KSbF₆, CF₃COOK, CH₃COOK, KPF₆, KNO₃, KClO₄, KSCN, KBPh₄, KB(C₆F₅)₄, KB(3,5-(CF₃)₂C₆F₃)₄, LiOTf, LiBF₄, LiSbF₆, CF₃COOLi, CH₃COOLi, LiPF₆, LiNO₃, LiClO₄, LiSCN, LiBPh₄, LiB(C₆F₅)₄, LiB(3,5-(CF₃)₂C₆F₃)₄, and the like, but are not particularly limited thereto. For example, AgBF₄ is easy to use.

The amount of the metal salt G-Q used is equimolar or more relative to ruthenium atoms.

Solvents used are not particularly limited, and include alcohols such as methanol, ethanol, and isopropanol, aromatic hydrocarbons such as toluene and xylene, halogenated hydrocarbons such as dichloromethane and 1,2-dichloroethane, aprotic polar solvents such as acetonitrile and N,N-dimethylformamide, ethers such as diethyl ether and tetrahydrofuran, and the like, of which methanol and dichloromethane are preferable. A single solvent may be used, or multiple solvents may be used as a mixture.

The amount of the solvent used is not particularly limited, and is, for example, 2 times by volume to 20 times by volume relative to the substrate (solvent mL/substrate g).

The reaction temperature is not particularly limited, and is 0°C to a temperature not higher than the boiling point of the solvent, and preferably room temperature to 80°C.

The reaction time varies depending on the reaction substrate used, and is 30 minutes to 20 hours, and preferably 1 hour to 12 hours. This production method is preferably conducted in an inert gas such as nitrogen gas or argon gas.

Note that when the complex represented by general formula (2) is used for a reduction reaction, an isolated complex of general formula (2) may be used for the reduction reaction, or the reduction reaction may be conducted, while synthesizing the complex of general formula (2) in the reduction reaction system according to scheme 8 (in situ method).

After preparation of the complex, the target solid-supported ruthenium complex can be separated by a common crystallization technique such as concentration of the reaction liquid or addition of a poor solvent.

In addition, if a metal halide salt (G-X) is by-produced in the above-described complex preparation, an operation of washing with water may be conducted, if necessary.

### <Reduction Reactions>

Reduction products can be produced by reducing organic compounds in the presence of a hydrogen donor by using any of the above-described solid-supported ruthenium complexes as a catalyst.

The reduction reactions of the present invention include an alcohol production method comprising reducing a carbonyl group of a carbonyl compound and an amine production method comprising reducing an imino group of an imine compound, both of which use the above-described solid-supported ruthenium complex as a catalyst and are carried out in the presence of a hydrogen donor. In addition, when the above-described solid-supported ruthenium complex is in an optically active form, an optically active alcohol can be produced by asymmetric reduction of a carbonyl group of a carbonyl compound, or an optically active amine can be produced by reduction of an imino group of an imine compound.

The hydrogen donor is not particularly limited, as long as the hydrogen donor is commonly used one for hydrogen transfer reduction reaction, such as formic acid, an alkali metal salt thereof, or an alcohol having a hydrogen atom at the α-position of a hydroxy-bearing carbon atom, for example, isopropanol. It is also possible to use hydrogen gas as the hydrogen donor.

The reduction reaction is preferably conducted in the presence of a base. The bases include tertiary organic amines such as trimethylamine, triethylamine, triisopropylamine, 1,4-diazabicyclo[2,2,2]octane (DABCO), and 1,8-diazabicyclo[5,4,0]undec-7-ene (DBU); and inorganic bases such as LiOH, NaOH, KOH, and K₂CO₃. A preferred base is triethylamine. The base is used in an excessive amount relative to the solid-supported ruthenium complex, and, for example, the amount used is 1 to 100000 times in terms of the mole ratio. When triethylamine is used, the triethylamine is preferably used in an amount of 1 to 10000 times the solid-supported ruthenium complex.

Regarding the combination of the above-described hydrogen donor with the base, it is preferable to use an amine as the base, when the hydrogen donor is formic acid. In this case, formic acid and the amine may be added separately to the reaction system, or a mixture thereof may be added to the reaction system. When the mixture is used, formic acid and the amine can be used by being mixed at any ratio. In addition, it is possible to prepare an azeotrope of formic acid and an amine in advance, and then use the azeotrope. Preferred examples of the azeotrope of formic acid and an amine include a formic acid-triethylamine (5:2 (mole ratio)) azeotrope and the like.

Regarding the reaction solvent, the hydrogen donor itself can be used in general, when the hydrogen donor is liquid. It is also possible to use one of or a mixture of non-hydrogen-donating solvents such as toluene, tetrahydrofuran, acetonitrile, dimethylformamide, dimethyl sulfoxide, acetone, and methylene chloride, methanol, ethanol, and n-butanol. In a case where a formic acid salt is used or the like, it is also possible to conduct the reaction in a tow-layer system by using water as an auxiliary solvent for dissolving the formic acid salt in combination with an organic solvent. In this case, a phase-transfer catalyst may also be used to accelerate the reaction. In addition, when hydrogen gas is used as the hydrogen donor, alcohol solvents such as methanol, ethanol, isopropanol, trifluoroethanol, or hexafluoro-2-propanol are preferable.

The amount of each of the solid-supported ruthenium complexes of general formulae (1) to (3) serving as the catalyst is such that the mole ratio (S/C) of the substrate (a carbonyl compound or an imine) (S) to the ruthenium metal atoms (C) is selected from a range of 10 to 1000000, preferably 50 to 15000.

The amount of the hydrogen donor used is generally equimolar or more relative to the carbonyl compound or the imine. In particular, when the hydrogen donor is formic acid or a salt thereof, the amount is preferably 1.5 times by mole or more, and also the hydrogen donor is used in a range of 40 times by mole or less, and preferably 20 times by mole or less. From the viewpoint of molecular efficiency, the amount is preferably 2 to 15 times by mole. Meanwhile, when the hydrogen donor is isopropanol or the like, the hydrogen donor is used in a large excess relative to the substrate from the viewpoint of the reaction equilibrium, and is generally used in the range of 1000 times by mole or less.

The reaction temperature is selected from the range of -20 to 100°C, and preferably 0 to 70°C.

The reaction pressure is not particularly limited, and the reaction is conducted at generally 0.5 to 2 atm, and preferably normal pressure. In addition, when hydrogen gas is used, the reaction pressure is generally 5 MPa or lower.

The reaction time varies depending on the catalyst ratio, and is 1 to 100 hours, and generally 2 to 50 hours.

After completion of the reaction, the solid-supported ruthenium complex serving as the catalyst can be separated from the reaction liquid containing the reduced target product by a simple approach such as filtration of the reaction liquid. In addition, the solid-supported ruthenium complex separated by filtration or the like can be reused directly for the next reaction. The reaction liquid from which the catalyst has been removed can be subjected to ordinary operations such as distillation, extraction, chromatography, and recrystallization to separate and purify the produced product or optically active form.

In addition, as for the reduction reaction of the present invention, a continuous flow reaction can be carried out by packing the solid-supported ruthenium complex serving as a catalyst in a column, and feeding the substrate to the column. By feeding the substrate, the hydrogen donor, and the solvent to the column kept at the above-described reaction temperature using a metering pump such as an HPLC pump, a reduction product can be produced by reducing an organic compound, without the need for the catalyst separation operation. Reaction conditions are the same as described above.

The reaction operations are, for example, as follows: the reaction can be conducted by mixing the substrate, the solvent, the base, and the hydrogen donor, and feeding the mixture liquid to the column. Meanwhile, when the hydrogen donor is hydrogen gas, the reaction can be conducted by mixing the substrate, the solvent, and the base, feeding the mixture liquid to the column, and causing hydrogen gas to pass through the column.

Hereinafter, the present invention will be described in detail based on Examples; however, the present invention is not limited thereto.

### [Examples]

NMR spectra used to identify complexes of the following Examples and the like and to determine the purity thereof were measured on a Mercury Plus 300 4N model apparatus manufactured by Varian Technologies Japan, Ltd. or Bruker BioSpin Avance III 500 System. For GC analysis, Chirasil-DEX CB (0.25 mm × 25 m, 0.25 µm) (manufactured by Varian, Inc.) or HP-1 (0.32 mm × 30 m, 0.25 µm) (manufactured by Agilent Technologies, Inc.) was used. For MS measurement, JMS-T100GCV manufactured by JEOL Ltd. or LCMS-IT-TOF manufactured by Shimadzu Corporation were used.

In addition, symbols in Examples have the following meanings.
Ph: phenyl
DPEN: 1,2-diphenylethylenediamine
DIPEA: diisopropylethylamine
Ts: tosyl
Et₃N: triethylamine
Me: methyl
2-MeOEtOH: 2-methoxyethanol
PS: polystyrene
HFIP: 1,1,1,3,3,3-hexafluoro-2-propanol
THF: tetrahydrofuran

### [Example 1] Synthesis of

### (1R,2R)-N¹-(2-((4-Methylcyclohexa-1,4-dienyl)methoxy)ethyl)-1,2-diphenylethane-1,2-diamine (Compound 2)

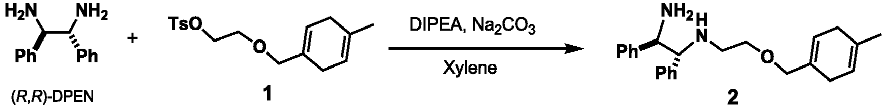

To a 300 mL 4-necked reaction flask, a three-way stopcock, a stir bar, and a thermometer were attached, and the inside was purged with nitrogen. To this reaction flask, 5.0 g (23.5 mmol) of (R,R)-DPEN, 30.38 g (18.8 mmol) of a 20% by weight toluene solution of compound 1, 4.99 g (47.1 mmol) of Na₂CO₃, 6.09 g (47.1 mmol) of DIPEA, and 100 mL of xylene were added under a nitrogen stream, and stirred for 11 hours in an oil bath of 140°C. After that, 60 mL of water and 50 mL of toluene were added, followed by stirring. Then, the mixture was allowed to stand, followed by liquid-liquid separation. Further, the organic layer was washed with 30 mL of water, and the organic layer was concentrated using an evaporator. Subsequent purification by silica gel column chromatography (ethyl acetate 100% to ethyl acetate:methanol=1:1 (volume ratio)) yielded 4.39 g of compound 2 (percentage yield: 64.3%).
¹H-NMR (CDCl₃, 300 MHz): δ 7.30-7.10 (m, 10H), 5.62 (m, 1H), 5.45 (m, 1H), 4.00 (d, 1H), 3.80 (m, 2H), 3.78 (d, 1H), 3.50-3.38 (m, 2H), 2.70-2.50 (m, 6H), 2.45-2.25 (brs, 3H), 1.70 (s, 3H);

### [Example 2] Synthesis of

### N-((1R,2R)-2-(2-((4-Methylcyclohexa-1,4-dienyl)methoxy)ethylamino)-1,2-diphenylet hyl)-4-(2-(trimethoxysilyl)ethyl)benzenesulfonamide (Compound 4)

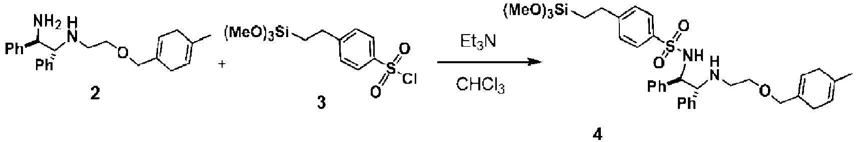

To a 300 mL 4-necked reaction flask, a three-way stopcock, a stir bar, and a thermometer were attached, and the inside was purged with nitrogen. To this reaction flask, 4.39 g (12.1 mmol) of compound 2 obtained in Example 1, 60 mL of chloroform, and 2.45 g (24.2 mmol) of triethylamine were added under a nitrogen stream, followed by stirring. Then, 7.86 g (12.1 mmol) of a 50% by weight methylene chloride solution of compound 3 was added dropwise. After stirring at room temperature for 2 hours, the reaction liquid was washed by adding water followed by stirring, and the obtained chloroform layer was subjected to an evaporator to remove the solvent. Purification by silica gel column chromatography (hexane:ethyl acetate=3:1 (volume ratio) to 2:1) yielded 4.0 g of compound 4 (percentage yield: 50.8%).
¹H-NMR (CDCl₃, 300 MHz): δ 7.40 (d, 2H), 7.20-6.85 (m, 8H), 5.63 (m, 1H), 5.43 (m, 1H), 4.41 (d, 1H), 3.90 (m, 1H), 3.80 (m, 1H), 3.59 (s, 9H), 3.60-3.40 (m, 2H), 3.75-3.60 (m, 2H), 3.60 (brs, 2H), 1.70 (s, 3H), 0.95-0.85 (m, 2H);

### [Example 3] Synthesis of Ruthenium Dimer Complex (Compound 5)

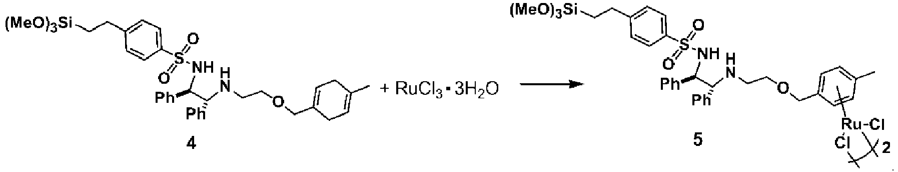

To a 100 mL recovery flask, 0.26 g (0.4 mmol) of compound 4 obtained in Example 2, 70 mg (0.267 mmol) of RuCl₃ · 3H₂O, and 7 mL of 2-MeOEtOH were added, followed by purging with nitrogen. Then, the mixture was stirred for 1 hour in an oil bath of 110°C. The reaction liquid turned red, indicating that complexing of ruthenium occurred.

### [Example 4] Synthesis of Silica Gel-Supported Ruthenium Dimer Complex (Compound 6A) (Solid-Supported Ruthenium Complex of Formula (3))

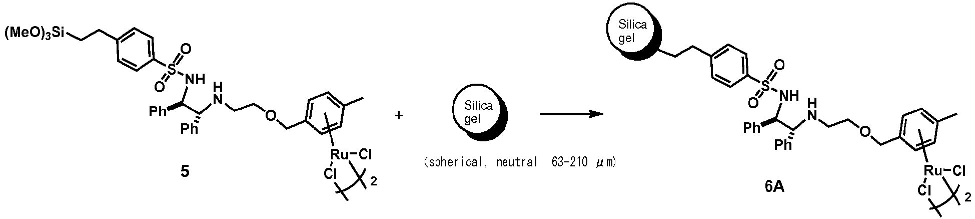

To a 2-MeOEtOH solution of compound 5 obtained in Example 3, 7.6 g of silica gel (manufactured by Kanto Chemical Co., Inc., neutral, spherical, 63 to 210 µm) was added, followed by stirring. Then, the solvent was recovered by using an evaporator. A portion (3 g) of the obtained sand-like solid was placed in a 200 mL recovery flask equipped with a Dean-Sterk reflux condenser, and 50 mL of toluene was added. The mixture was heated for 2 hours in an oil bath of 140°C, while removing the distilled water and methanol. After that, the mixture was cooled to normal temperature, and the solid was collected by filtration using a Kiriyama-funnel. Then, the solid was washed with 30 mL of toluene, and subsequently 30 mL of methanol. Drying the obtained solid under reduced pressure yielded a silica gel-supported ruthenium dimer complex (compound 6A) of the present invention.

### [Example 5] Reduction Reaction of Acetophenone using Silica Gel-Supported Ruthenium Dimer Complex (Compound 6A)

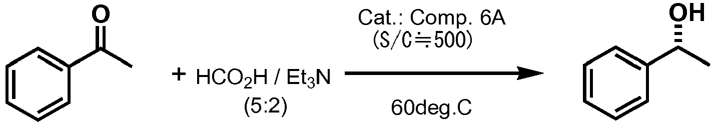

To a 15 mL Schlenk tube, 0.41 g (0.01 mmol in terms of monomer complex) of compound 6A obtained in Example 4, 0.6 g (5.0 mmol) of acetophenone, and 2.5 mL of a formic acid-triethylamine (5:2 (mole ratio)) azeotrope were added, followed by purging with nitrogen. Then, the reaction was started at 60°C. The amount of moles of formic acid was 9 times that of acetophenone.

The conversion and the optical purity were measured by GC after reaction times of 1 hour, 2 hours, and 5 hours. The results were as shown in Table 1.

After completion of the reaction, 5 mL of ethyl acetate was added to the solution, and then the solid catalyst was collected by filtration using a Kiriyama-funnel to recover the solid-supported ruthenium complex of the present invention.

A second reaction was conducted by using the recovered solid-supported ruthenium complex by the same operations as in the first reaction. The conversions and the optical purities after reaction times of 1 hour, 2 hours, and 5 hours were as shown in Table 1. After completion of the reaction, the solid-supported ruthenium complex of the present invention was recovered by the same operation as in the first reaction.

A third reaction was conducted by using the solid-supported ruthenium complex recovered after the use thereof for the second reaction and by the same operations as in the first reaction. The conversions and the optical purities after reaction times of 1 hour, 2 hours, and 7 hours were as shown in Table 1.

**[Table 1]**

| | | Reaction time (hours) | Conversion (%) | Optical purity (% ee) |
|---|---|---|---|---|
| Experiment (run) 1 | First reaction | 1 | 18.9 | 96.3 |
| | | 2 | 44.8 | 96.7 |
| | | 5 | 91.1 | 97.1 |
| Experiment (run) 2 | Second reaction (reuse reaction after first recovery) | 1 | 22.4 | 96.2 |
| | | 2 | 45.1 | 96.2 |
| | | 5 | 92.1 | 96.8 |
| Experiment (run) 3 | Third reaction (reuse reaction after second recovery) | 1 | 16.0 | 94.7 |
| | | 2 | 31.7 | 96.2 |
| | | 7 | 90.5 | 96.9 |

As described above, with a catalytic amount of S/C=approximately 500, the silica gel-supported ruthenium complex of the present invention achieved a conversion exceeding 90% also after 7 hours in the third reaction, which was conducted after the second recovery. Accordingly, it was found that the ruthenium complex was firmly supported on the solid support in the solid-supported ruthenium complex of the present invention, and the solid-supported ruthenium complex retained a high catalytic activity in the reuse after recovery.

### [Example 6] Synthesis of Silica Gel-Supported Ruthenium Dimer Complex (Compound 6B) (Solid-Supported Ruthenium Complex of Formula (3))

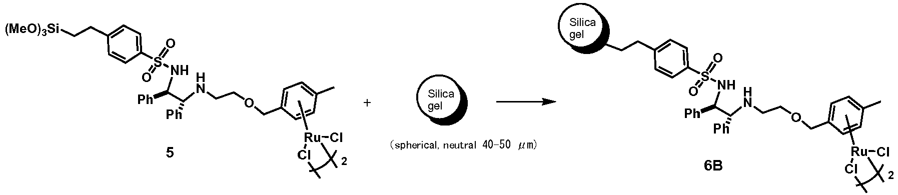

To a 2-MeOEtOH solution of compound 5 obtained in Example 3, 7.6 g of silica gel (manufactured by Kanto Chemical Co., Inc., neutral, spherical, 40 to 50 µm) was added, followed by stirring. Then, the solvent was recovered by using an evaporator. A portion (3 g) of the obtained sand-like solid was placed in a 200 mL recovery flask equipped with a Dean-Sterk reflux condenser, and 50 mL of toluene was added. Then, the mixture was heated for 2 hours in an oil bath of 140°C, while removing the distilled water and methanol. After that, the mixture was cooled to normal temperature, and the solid was collected by filtration using a Kiriyama-funnel. Then, the solid was washed with 30 mL of toluene, and subsequently 30 mL of methanol. Drying the obtained solid under reduced pressure yielded a silica gel-supported ruthenium dimer complex (compound 6B) of the present invention.

### [Example 7] Reduction Reaction of Acetophenone using Silica Gel-Supported Ruthenium Dimer Complex (Compound 6B)

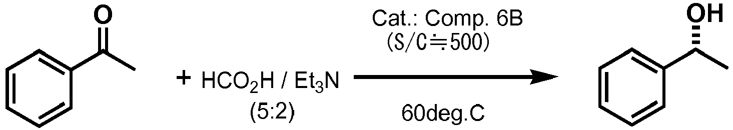

Reactions and catalyst recovery were conducted in the same manner as in Example 5, except that compound 6B obtained in Example 6 was used, and that the reaction time was changed as shown in Table 2 below.

**[Table 2]**

| | | Reaction time (hours) | Conversion (%) | Optical purity (% ee) |
|---|---|---|---|---|
| Experiment (run) 1 | First reaction | 1 | 37.9 | 95.9 |
| | | 2 | 72.0 | 96.7 |
| | | 3 | 90.2 | 96.7 |
| Experiment (run) 2 | Second reaction (reuse reaction after first recovery) | 1 | 32.0 | 95.6 |
| | | 2 | 63.6 | 96.6 |
| | | 4 | 93.4 | 96.8 |
| Experiment (run) 3 | Third reaction (reuse reaction after second recovery) | 1 | 26.6 | 95.7 |
| | | 4 | 89.0 | 96.8 |
| | | 5 | 94.9 | 96.8 |

With a catalytic amount of S/C=approximately 500, the silica gel-supported ruthenium complex of the present invention achieved a conversion exceeding 90% also after 5 hours in the third reaction, which was conducted after the second recovery. Accordingly, it was found that the ruthenium complex was firmly supported on the solid support in the solid-supported ruthenium complex of the present invention, and that the solid-supported ruthenium complex of the present invention retained a high catalytic activity when reused after the recovery.

### [Example 8] Synthesis of Compound 8A

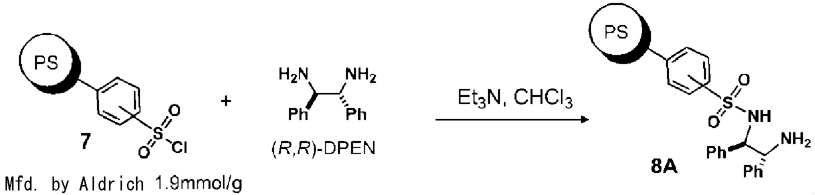

To a 100 mL Schlenk tube, 1.0 g (1.9 mmol) of sulfonyl chloride functional group-bearing polystyrene beads (compound 7) (manufactured by Aldrich, 1.9 mmol/g), 0.807 g (3.8 mmol) of (R,R)-DPEN, 0.77 g (7.6 mmol) of triethylamine, and 10 mL of chloroform were added, followed by purging with nitrogen. Then, the mixture was stirred in an oil bath of 50°C for 2.5 hours. After that, the reaction liquid was cooled to normal temperature, and filtered using a Kiriyama-funnel. The solid was washed with chloroform, and dried under reduced pressure. Thus, 1.33 g of compound 8A was obtained (percentage yield: 100% (in terms of weight)).

### [Example 9] Synthesis of Compound 9A

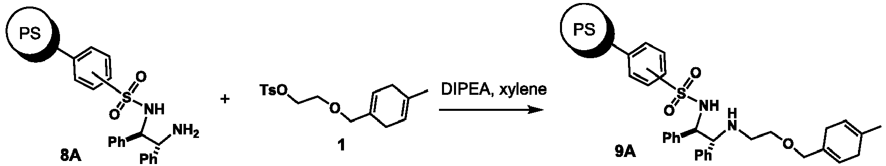

To a 100 mL Schlenk tube, 0.5 g (0.95 mmol) of compound 8A obtained in Example 8, 1.91 g (1.187 mmol) of a 20% by weight toluene solution of compound 1, 0.245 g (1.9 mmol) of DIPEA, and 10 mL of xylene were added, and stirred for 9 hours in an oil bath of 140°C. After that, the reaction liquid was cooled to normal temperature, and filtered using a Kiriyama-funnel. The solid was washed with 10 mL of toluene and 10 mL of chloroform, and dried under reduced pressure. Thus, 0.66 g of compound 9A was obtained (percentage yield: 99% (in terms of weight)).

### [Example 10] Synthesis of Polystyrene-Supported Ruthenium Dimer Complex (Compound 10A) (Solid-Supported Ruthenium Complex of Formula (3))

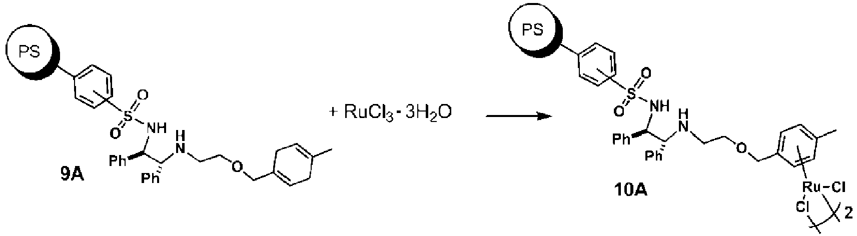

To a 50 mL recovery flask, 0.2 g (0.38 mmol) of compound 9A obtained in Example 9, 99.35 mg (0.38 mmol) of RuCl₃·3H₂O, 5 mL of 2-MeOEtOH, and 1 mL of water were added, and the inside was purged with nitrogen. Then, the reaction was allowed to proceed for 2 hours in an oil bath of 110°C. The reaction liquid was cooled to room temperature, and filtered using a Kiriyama-funnel. The obtained solid was washed with 20 mL of methanol, and then dried under reduced pressure. Thus, 0.211 g of compound 10A was obtained, which is a solid-supported ruthenium complex of the present invention.

### [Example 11] Reduction Reaction of Acetophenone using Polystyrene-Supported Ruthenium Dimer Complex (Compound 10A)

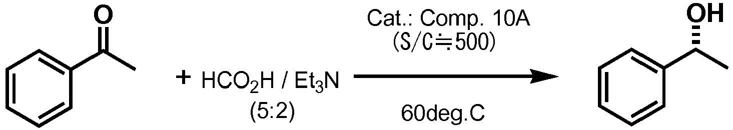

To a 15 mL Schlenk tube, 13.15 mg (0.005 mmol in terms of monomer complex) of compound 10A obtained in Example 10, 0.3 g (2.5 mmol) of acetophenone, and 1.25 mL of a formic acid-triethylamine (5:2 (mole ratio)) azeotrope were added, followed by purging with nitrogen. Then, the reaction was started at 60°C. The amount of moles of formic acid was 9 times that of acetophenone. After 2 hours of the reaction, the conversion and the optical purity were measured. The conversion was 20.4%, and the optical purity was 94.3% ee. The reaction was further continued, and the conversion and the optical purity were measured at a reaction time of 9 hours. The conversion was 93.8%, and the optical purity was 95.7% ee.

### [Example 12] Synthesis of Compound 9B

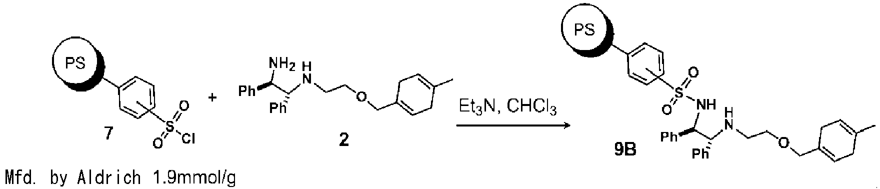

To a 50 mL recovery flask, 0.5 g (0.95 mmol) of sulfonyl chloride functional group-bearing polystyrene beads (compound 7) (manufactured by Aldrich, 1.9 mmol/g), 0.516 g (1.425 mmol) of compound 2 obtained in Example 1, 0.29 g (2.85 mmol) of triethylamine, and 20 mL of chloroform were added, followed by purging with nitrogen. Then, the mixture was stirred for 2 hours in an oil bath of 50°C. After that, the reaction liquid was cooled to normal temperature, and filtered using a Kiriyama-funnel. Then, the solid was washed with chloroform, and dried under reduced pressure. Thus, 0.935 g of compound 9B was obtained.

### [Example 13] Synthesis of Polystyrene-Supported Ruthenium Dimer Complex (Compound 10B) (Solid-Supported Ruthenium Complex of Formula (3))

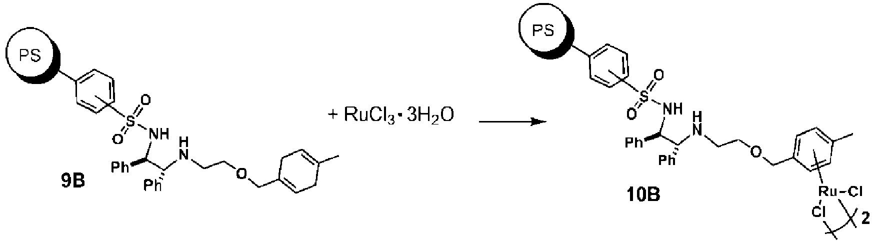

To a 50 mL recovery flask, 0.2 g (0.38 mmol) of compound 9B obtained in Example 12, 99.35 mg (0.38 mmol) of RuCl₃·3H₂O, and 5 mL of 2-MeOEtOH were added, and then the inside was purged with nitrogen. Then, the reaction was allowed to proceed for 2 hours in an oil bath of 110°C. The reaction liquid was cooled to room temperature, and filtered using a Kiriyama-funnel. The obtained solid was washed with 20 mL of methanol, and then dried under reduced pressure. Thus, 0.240 g of compound 10B was obtained, which is a solid-supported ruthenium complex of the present invention.

### [Example 14] Reduction Reaction of Acetophenone using Polystyrene-Supported Ruthenium Dimer Complex (Compound 10B)

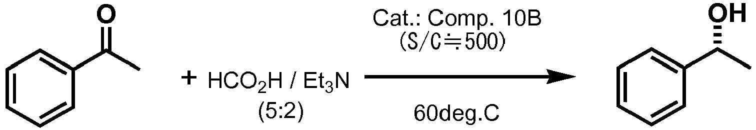

To a 15 mL Schlenk tube, 13.2 mg (0.005 mmol in terms of monomer complex) of compound 10B obtained in Example 13, 0.3 g (2.5 mmol) of acetophenone, and 1.25 mL of a formic acid-triethylamine (5:2 (mole ratio)) azeotrope were added, followed by purging with nitrogen. Then, the reaction was started at 60°C. The amount of moles of formic acid was 9 times that of acetophenone.

The conversion and the optical purity after reaction times of 3 hours and 5 hours were as shown in Table 3 below.

After completion of the reaction, 5 mL of ethyl acetate was added to the solution, and then the supernatant of the reaction liquid was taken out with a syringe. A second reaction was conducted by adding a fresh substrate and a fresh formic acid-triethylamine (5:2) azeotrope to the solid catalyst remaining in the Schlenk tube in the same manner as in the first reaction. The conversion and the optical purity after reaction times of 1 hour and 3 hours were as shown in Table 3 below.

After completion of the reaction, the reaction liquid was taken out by the same operation as in the first reaction, and a third reaction was conducted by adding a fresh substrate and a fresh formic acid-triethylamine azeotrope to the solid catalyst remaining in the Schlenk tube in the same manner as in the first reaction. The conversion and the optical purity after reaction times of 1 hour and 4 hours were as shown in Table 3 below.

**[Table 3]**

| | | Reaction time (hours) | Conversion (%) | Optical purity (% ee) |
|---|---|---|---|---|
| Experiment (run) 1 | First reaction | 3 | 50.1 | 97.4 |
| | | 5 | 95.3 | 97.8 |
| Experiment (run) 2 | Second reaction (reuse reaction after first recovery) | 1 | 34.7 | 97.9 |
| | | 3 | 94.8 | 98.0 |
| Experiment (run) 3 | Third reaction (reuse reaction after second recovery) | 1 | 22.4 | 97.2 |
| | | 4 | 94.4 | 97.6 |

With a catalytic amount of S/C=approximately 500, the silica gel-supported ruthenium complex of the present invention achieved a conversion exceeding 90% also after 4 hours in the third reaction, which was conducted after the second recovery. Accordingly, it was found that the ruthenium complex was firmly supported on the solid support in the solid-supported ruthenium complex of the present invention, and that the solid-supported ruthenium complex of the present invention retained a high catalytic activity when reused after the recovery.

### [Example 15] Synthesis of

### (1R,2R)-N¹-(5-(4-Methylcyclohexa-1,4-dienyl)pentyl)-1,2-diphenylethane-1,2-diamine (Compound 12)

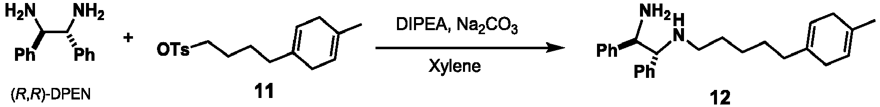

To a 200 mL 4-necked reaction flask, a three-way stopcock, a stir bar, and a thermometer were attached, and the inside was purged with nitrogen. To this reaction flask, 10.0 g (47.1 mmol) of (R,R)-DPEN, 6.04 g (18.8 mmol) of compound 11, 9.98 g (94.2 mmol) of Na₂CO₃, 12.17 g (94.2 mmol) of DIPEA, and 200 mL of xylene were added under a nitrogen stream, and stirred for 10 hours in an oil bath of 140°C. After that, the reaction liquid was concentrated using an evaporator, and subsequent purification by silica gel column chromatography (hexane:ethyl acetate=1:1 (volume ratio) to ethyl acetate:methanol=10:1) yielded 3.01 g of compound 12 (percentage yield: 44.3%).
¹H-NMR (CDCl₃, 300 MHz): δ 7.38-7.10 (m, 10H), 5.60-5.30 (m, 2H), 4.22-4.04 (m, 1H), 3.85-3.70 (m, 1H), 2.78-2.16 (m, 9H), 2.08 (s, 1H), 2.03-1.92 (m, 1H), 1.90 (t, 2H), 1.78 (s, 1H), 1.65 (s, 2H) 1.58-1.20 (m, 4H);

### [Example 16] Synthesis of

### N-((1R,2R)-2-(5-(4-Methylcyclohexa-1,4-dienyl)pentylamino)-1,2-diphenylethyl)-4-(2-(trimethoxysilyl)ethyl)benzenesulfonamide (Compound 13)

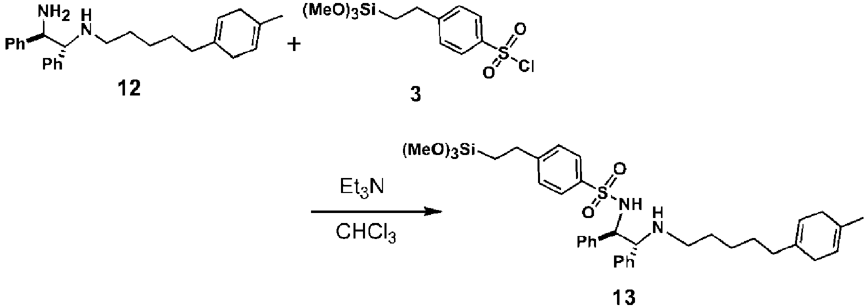

To a 100 mL 4-necked reaction flask, a three-way stopcock, a stir bar, and a thermometer were attached, and the inside was purged with nitrogen. To this reaction flask, 1.0 g (2.77 mmol) of compound 12, 14 mL of chloroform, and 0.56 g (5.54 mmol) of triethylamine were added under a nitrogen stream followed by stirring. Then, 1.98 g (3.05 mmol) of a 50% by weight methylene chloride solution of compound 3 was added dropwise. After stirring at room temperature for 2 hours, the mixture was washed by adding water followed by stirring, and the obtained chloroform layer was subjected to an evaporator to remove the solvent. Purification by silica gel column chromatography (hexane:ethyl acetate=4:1) yielded 0.72 g of compound 13 (percentage yield: 40.1 %).
¹H-NMR (CDCl₃, 300 MHz): δ 7.62 (d, 1H), 7.50-6.78 (m, 14H), 5.56-5.20 (m, 2H), (m, 1H), 3.60 (s, 9H), 3.10-2.18 (m, 9H), 2.10-1.56 (m, 9H), 1.45-1.20 (m, 4H), 1.05-0.80 (m, 2H);

### [Example 17] Synthesis of Ruthenium Dimer Complex (Compound 14)

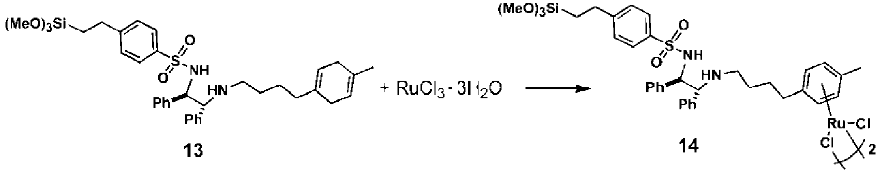

To a 100 mL side-arm flask, 0.24 g (0.37 mmol) of compound 13 obtained in Example 16, 65.6 mg (0.251 mmol) of RuCl₃·3H₂O, and 7 mL of 2-MeOEtOH were added, followed by purging with nitrogen. Then, the mixture was stirred in an oil bath of 110°C for 2 hours. The reaction liquid turned red, indicating that complexing of ruthenium occurred.

### [Example 18] Synthesis of Silica Gel-Supported Ruthenium Dimer Complex (Compound 15A) (Solid-Supported Ruthenium Complex of Formula (3))

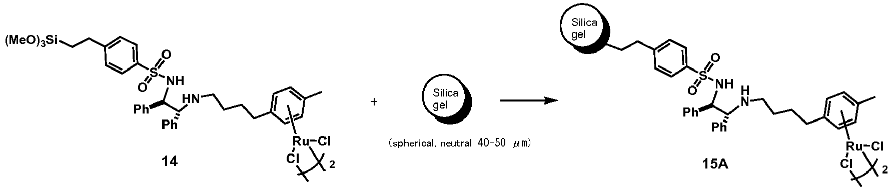

To a 2-MeOEtOH solution of compound 14 obtained in Example 17, 10.28 g of silica gel (manufactured by Kanto Chemical Co., Inc., neutral, spherical, 40 to 50 µm) was added, followed by stirring. Then, the solvent was recovered by using an evaporator. A portion (3 g) of the obtained sand-like solid was placed in a 200 mL recovery flask equipped with a Dean-Sterk reflux condenser, and 50 mL of toluene was added. Then, the mixture was heated for 2 hours in an oil bath of 140°C, while removing the distilled water and methanol. After that, the mixture was cooled to normal temperature, and the solid was collected by filtration using a Kiriyama-funnel. Then, the solid was washed with 30 mL of toluene, and subsequently 30 mL of methanol. Drying the obtained solid under reduced pressure yielded a silica gel-supported ruthenium dimer complex (compound 15A) of the present invention.

### [Example 19] Reduction Reaction of Acetophenone using Silica Gel-Supported Ruthenium Dimer Complex (Compound 15A)

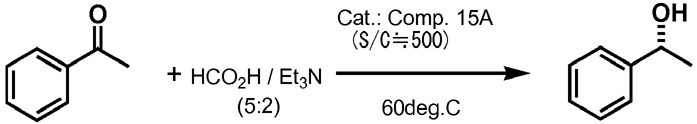

Reactions and catalyst recovery were conducted in the same manner as in Example 5, except that compound 15A obtained in Example 18 was used, and that the reaction time was changed as shown in Table 4 below.

**[Table 4]**

| | | Reaction time (hours) | Conversion (%) | Optical purity (% ee) |
|---|---|---|---|---|
| Experiment (run) 1 | First reaction | 1 | 32.4 | 97.0 |
| | | 3 | 97.1 | 98.3 |
| Experiment (run) 2 | Second reaction (reuse reaction after first recovery) | 1 | 42.9 | 97.3 |
| | | 3 | 93.0 | 97.4 |
| Experiment (run) 3 | Third reaction (reuse reaction after second recovery) | 3 | 81.3 | 97.3 |
| | | 5 | 98.2 | 98.3 |

With a catalytic amount of S/C=approximately 500, the silica gel-supported complex of the present invention achieved a conversion exceeding 90% also after 5 hours in the third reaction, which was conducted after the second recovery. Accordingly, it was found that the ruthenium complex was firmly supported on the solid support in the solid-supported ruthenium complex of the present invention, and that the solid-supported ruthenium complex of the present invention retained a high catalytic activity when reused after the recovery.

### [Example 20] Synthesis of Silica Gel-Supported Ruthenium Monomer Complex (Compound 6B') (Solid-Supported Ruthenium Complex of Formula (1))

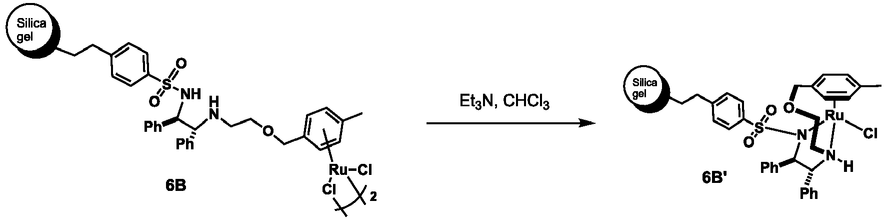

In a 100 mL recovery flask, 1.0 g (0.012 mmol in terms of monomer complex) of compound 6B obtained in Example 6 was placed, and 0.26 g (0.1 mmol) of triethylamine and 10 mL of chloroform were added. Then, the inside was purged with nitrogen, followed by stirring for 2 hours in an oil bath of 40°C. After that, the reaction liquid was cooled to normal temperature. The solid was collected by filtration using a Kiriyama-funnel, and washed with 30 mL of chloroform and subsequently 30 mL of methanol. Drying the obtained solid under reduced pressure yielded a silica gel-supported ruthenium monomer complex (compound 6B') of the present invention.

### [Example 21] Reduction Reaction of Acetophenone using Silica Gel-Supported Ruthenium Monomer Complex (Compound 6B')

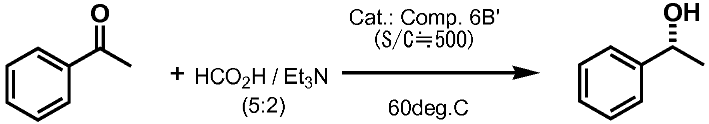

To a 15 mL Schlenk tube, 0.41 g (0.01 mmol) of compound 6B' obtained in Example 20, 0.6 g (5.0 mmol) of acetophenone, and 2.5 mL of a formic acid-triethylamine (5:2 (mole ratio)) azeotrope were added, followed by purging with nitrogen. Then, the reaction was allowed to proceed at 60°C for 5 hours. The amount of moles of formic acid was 9 times that of acetophenone. The conversion and the optical purity were measured by GC and found to be 97.8% conv. and 96.8% ee, respectively.

### [Example 22] Synthesis of Polystyrene-Supported Ruthenium Monomer Complex (Compound 10B') (Solid-Supported Ruthenium Complex of Formula (1))

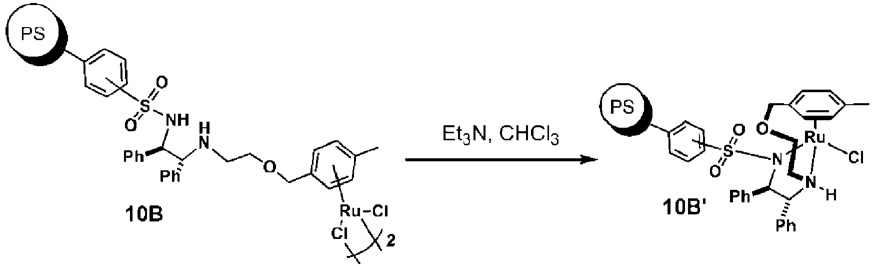

In a 100 mL recovery flask, 0.132 g (0.05 mmol in terms of monomer complex) of compound 10B obtained in Example 13 was placed, and 0.26 g (0.1 mmol) of triethylamine and 5 mL of chloroform were added. After the inside was purged with nitrogen, the mixture was stirred for 2 hours in an oil bath of 40°C. After that, the mixture was cooled to normal temperature. The solid was collected by filtration using a Kiriyama-funnel, and washed with 10 mL of chloroform and subsequently 10 mL of methanol. Drying the obtained solid under reduced pressure yielded a polystyrene-supported ruthenium monomer complex (compound 10B') of the present invention.

### [Example 23] Reduction Reaction of Acetophenone using Polystyrene-Supported Ruthenium Monomer Complex (Compound 10B')

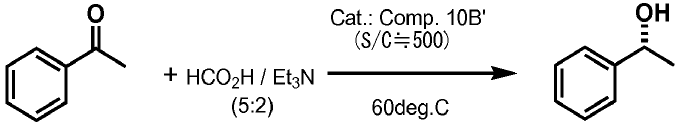

To a 15 mL Schlenk tube, 13.2 mg (0.005 mmol) of compound 10B' obtained in Example 22, 0.3 g (2.5 mmol) of acetophenone, and 1.25 mL of a formic acid-triethylamine (5:2 (mole ratio)) azeotrope were added, followed by purging with nitrogen. Then, the reaction was allowed to proceed at 60°C for 5 hours. The amount of moles of formic acid was 9 times that of acetophenone. The conversion and the optical purity were measured by GC and found to be 96.5% conv. and 97.8% ee, respectively.

### [Example 24] Reduction Reaction of Propiophenone using Silica Gel-Supported Ruthenium Dimer Complex (Compound 6B)

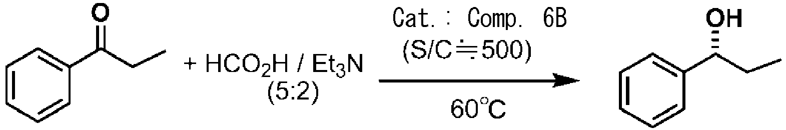

To a 15 mL Schlenk tube, 0.205 g (0.005 mmol in terms of monomer complex) of compound 6B obtained in Example 6, 0.335 g (2.5 mmol) of propiophenone, and 1.25 mL of a formic acid-triethylamine (5:2 (mole ratio)) azeotrope were added, followed by purging with nitrogen. Then, the reaction was started at 60°C. The amount of moles of formic acid was 9 times that of the substrate.

After a reaction time of 10 hours, the conversion and the optical purity were measured by GC and found to be 97.6% conv. and 96.9% ee, respectively.

### [Example 25] Reduction Reaction of Acetylfuran using Silica Gel-Supported Ruthenium Dimer Complex (Compound 6B)

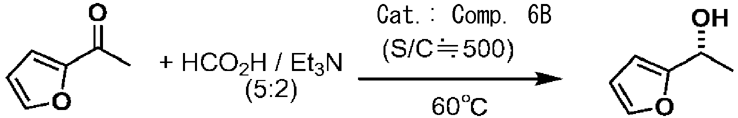

To a 15 mL Schlenk tube, 0.205 g (0.005 mmol in terms of monomer complex) of compound 6B obtained in Example 6, 0.275 g (2.5 mmol) of acetylfuran, and 1.25 mL of a formic acid-triethylamine (5:2 (mole ratio)) azeotrope were added, followed by purging with nitrogen. Then, the reaction was started at 60°C. The amount of moles of formic acid was 9 times that of the substrate.

After a reaction time of 5 hours, the conversion and the optical purity were measured by GC and found to be 100% conv. and 98.4% ee, respectively.

### [Example 26] Reduction Reaction of o-Methoxyacetophenone using Silica Gel-Supported Ruthenium Dimer Complex (Compound 6B)

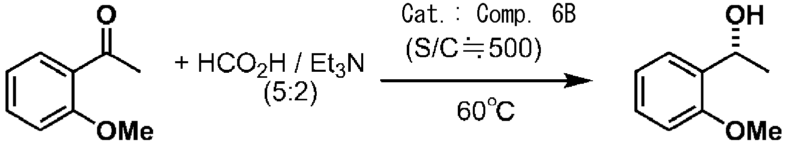

To a 15 mL Schlenk tube, 0.205 g (0.005 mmol in terms of monomer complex) of compound 6B obtained in Example 6, 0.375 g (2.5 mmol) of o-methoxyacetophenone, and 1.25 mL of a formic acid-triethylamine (5:2 (mole ratio)) azeotrope were added, followed by purging with nitrogen. Then, the reaction was started at 60°C. The amount of moles of formic acid was 9 times that of the substrate.

After a reaction time of 10 hours, the conversion and the optical purity were measured by GC and found to be 96.0% conv. and 94.1 % ee, respectively.

### [Example 27] Hydrogenation Reduction Reaction of α-Hydroxyacetophenone using Silica Gel-Supported Ruthenium Dimer Complex (Compound 6B)

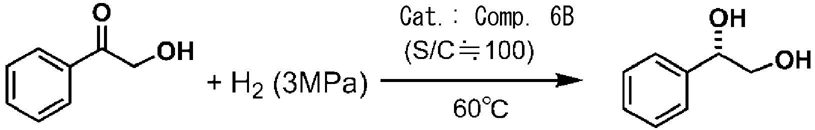

To a 100 mL autoclave, 0.205 g (0.005 mmol in terms of monomer complex) of compound 6B obtained in Example 6 and 0.07 g (0.5 mmol) of α-hydroxyacetophenone were added, followed by purging with nitrogen. Then, 1.4 mL of methanol was added. The pressure was raised to 3.0 MPa with hydrogen gas, and the reaction was started at 60°C.

After a reaction time of 8 hours, the conversion and the optical purity were measured by GC and found to be 97.3% conv. and 82% ee, respectively.

### [Example 28] Reduction Reaction of Cyclic Imine using Silica Gel-Supported Ruthenium Dimer Complex (Compound 6B)

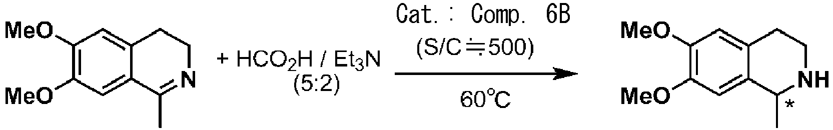

To a 15 mL Schlenk tube, 0.205 g (0.005 mmol in terms of monomer complex) of compound 6B obtained in Example 6, 0.102 g (0.5 mmol) of a cyclic imine represented by the above-described formula, 1.25 mL of a formic acid-triethylamine (5:2 (mole ratio)) azeotrope, and 3 mL of dichloromethane were added, followed by purging with nitrogen. Then, the reaction was started at 30°C. The amount of moles of formic acid was 9 times that of the cyclic imine.

After a reaction time of 20 hours, the conversion and the optical purity were measured by GC and found to be 98.0% conv. and 90% ee, respectively.

### [Example 29] Synthesis of Silica Gel-Supported Ruthenium Cationic Monomer Complex (Compound 6B'-BF₄) (Solid-Supported Ruthenium Complex of Formula (2))

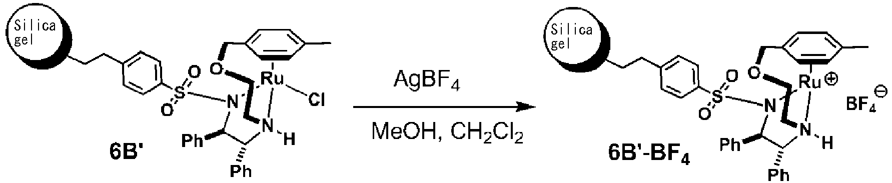

In a 100 mL recovery flask, 1.0 g (0.012 mmol) of compound 6B' obtained in Example 20 was placed, and 9.3 mg (0.048 mmol) of AgBF₄, 3 mL of methanol, and 5 mL of dichloromethane were added. After the inside was purged with nitrogen, the mixture was stirred for 2 hours in an oil bath of 40°C. After that, the reaction liquid was cooled to normal temperature. The solid was collected by filtration using a Kiriyama-funnel, and washed with 30 mL of chloroform, and subsequently 30 mL of methanol. Drying the obtained solid under reduced pressure yielded a silica gel-supported ruthenium cationic monomer complex (compound 6B'-BF₄) of the present invention.

### [Example 30] Hydrogenation Reduction Reaction of 2-Methylquinoline using Silica Gel-Supported Ruthenium Cationic Monomer Complex (Compound 6B'-BF₄)

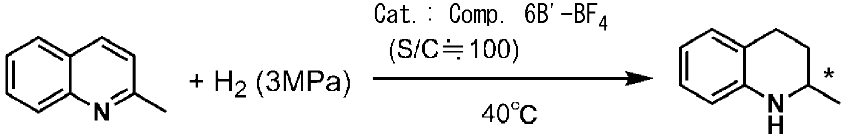

To a 100 mL autoclave, 0.205 g (0.005 mmol) of compound 6B'-BF₄ obtained in Example 29 and 0.07 g (0.5 mmol) of 2-methylquinoline were added, followed by purging with nitrogen. Then, 1.4 mL of HFIP was added. The pressure was raised to 3.0 MPa with hydrogen gas, and the reaction was started at 40°C.

After a reaction time of 20 hours, the conversion and the optical purity were measured by GC and found to be 95.0% conv. and 91 % ee, respectively.

### [Example 31] Synthesis of

### 4-(N-((1 R,2R)-2-((2-((4-Methylcyclohexa-1,4-dien-1 -yl)methoxy)ethyl)amino)-1,2-dip henylethyl)sulfamoyl)benzoic Acid (Compound 16)

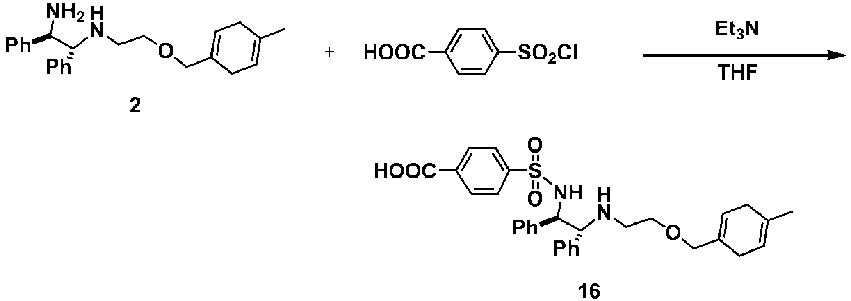

To a 30 mL recovery flask, 61 mg (0.28 mmol) of 4-chlorosulfonylbenzoic acid was added, followed by purging with nitrogen. Then, 56 mg (0.56 mmol) of triethylamine dissolved in 2 mL of THF was added dropwise. With stirring, this mixture was cooled to 0°C with ice water, and 100 mg (0.28 mmol, synthesized in Example 1) of compound 2 dissolved in 2 mL of THF was added dropwise, and the reaction was allowed to proceed. After 30 minutes, the reaction was stopped, and purification was conducted by silica gel chromatography (chloroform:methanol=20:1 (volume ratio)). Thus, 420 mg (0.768 mmol, percentage yield: 28%) of compound 16 was obtained.
¹H-NMR (CDCl₃, 300 MHz): δ 8.25-7.90 (m, 14H), 6.40-6.20 (m, 1H), 5.82-4.98 (m, 2H), 4.88-4.70 (m, 1H), 4.00-3.18 (m, 5H), 2.96-2.40 (m, 4H), 2.22-1.86 (m, 2H), 1.78-1.40 (m, 4H), 0.08 (s, 1H);

### [Example 32] Synthesis of Polystyrene-Supported Ligand (Compound 17)

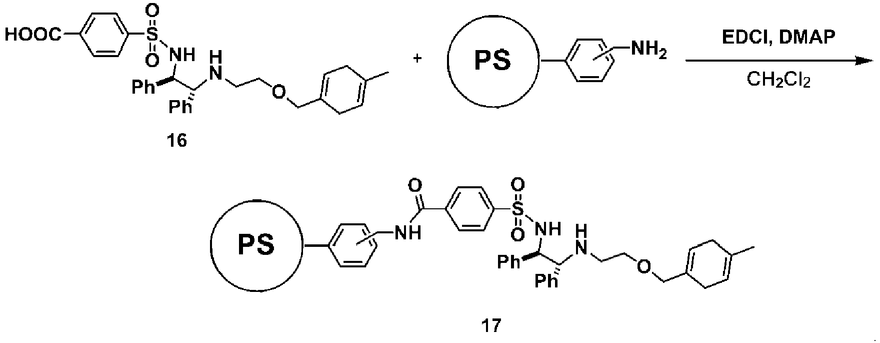

To a 30 mL recovery flask, 202 mg (0.37 mmol) of compound 16 obtained in Example 31, 85 mg (0.444 mmol) of 1-ethyl-3-[3-(dimethylamino)propyl]carbodiimide hydrochloride (abbreviated as EDCI), 54 mg (0.444 mmol) of dimethylaminopyridine (abbreviated as DMAP), and 1 mL of methylene chloride were added, followed by purging with nitrogen. After that, 1 mL of methylene chloride was further added, followed by stirring at room temperature for 20 hours. After the reaction was stopped, filtration was carried out on a Kiriyama-funnel, and the filtration residue was placed in a 30 mL recovery flask. After 10 mL of water and 10 mL of chloroform were added to the recovery flask, washing was carried out by stirring for 10 minutes. After that, filtration was carried out again, followed by washing with methanol and drying. Thus, 241 mg of compound 17 was obtained.

### [Example 33] Synthesis of Polystyrene-Supported Ruthenium Dimer Complex (Compound 18) (Solid-Supported Ruthenium Complex of Formula (3))

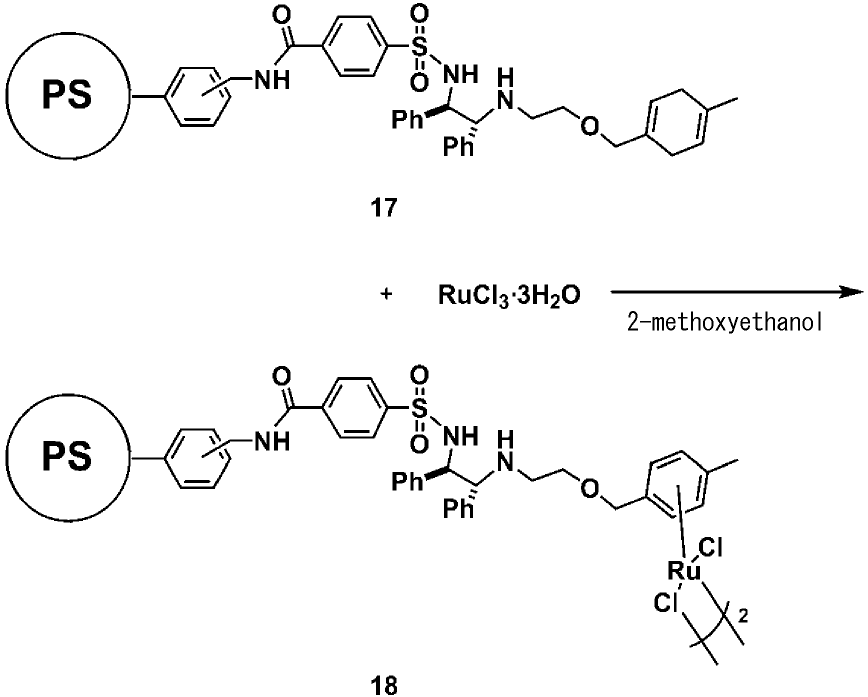

To a 30 mL recovery flask, 0.2 g (0.31 mmol) of compound 17 obtained in Example 32 and 80 mg (0.31 mmol) of RuCl₃·3H₂O were added, followed by purging with nitrogen. After that, 5 mL of 2-methoxyethanol was added, followed by stirring at 110°C for 2 hours. After the reaction was stopped, filtration was carried out on a Kiriyama-funnel, and the filtration residue was washed with methanol and then dried. Thus, 192 mg of a polystyrene-supported ruthenium dimer complex (compound 18) of the present invention was obtained.

### [Example 34] Reduction Reaction of Acetophenone using Polystyrene-Supported Ruthenium Dimer Complex (Compound 18)

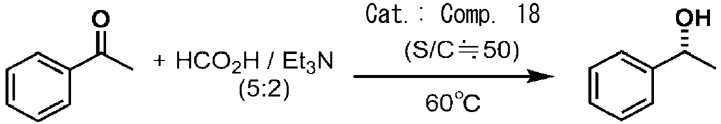

To a 20 mL Schlenk tube, 68.7 mg (0.1 mmol in terms of monomer complex) of compound 18 obtained in Example 33, 0.62 g (5 mmol) of acetophenone, and 2.5 mL of a formic acid-triethylamine (5:2 (mole ratio)) azeotrope were added, followed by purging with nitrogen. Then, the reaction was started at 60°C. The amount of moles of formic acid was 9 times that of acetophenone.

The conversions and the optical purities after reaction times of 5 hours and 7 hours were as shown in Table 5.

After completion of the reaction, 5 mL of ethyl acetate was added to the solution. Then, the solid catalyst was collected by filtration using a Kiriyama-funnel to recover the solid-supported ruthenium complex of the present invention.

A second reaction was conducted by using the recovered solid-supported ruthenium complex by the same operations as in the first reaction. The conversions and the optical purities after reaction times of 5 hours and 7 hours were as shown in Table 5.

**[Table 5]**

| | | Reaction time (hours) | Conversion (%) | Optical purity (ee %) |
|---|---|---|---|---|
| Experiment (run) 1 | First reaction | 5 | 80.6 | 97.5 |
| | | 7 | 95.3 | 96.6 |
| Experiment (run) 2 | Second reaction (reuse reaction after first recovery) | 5 | 47.6 | 98.2 |
| | | 7 | 62.4 | 98.5 |

With the polystyrene-supported complex of the present invention in a catalytic amount of S/C=approximately 50, the reaction proceeded and the optical purity exceeded 90% also after 5 hours of the second reaction, which was conducted after the first recovery. Accordingly, it was found that the ruthenium complex was firmly supported on the solid support in the solid-supported ruthenium complex of the present invention.

### [Example 35] Continuous Flow-Type Reduction Reaction of Acetophenone using Silica Gel-Supported Ruthenium Dimer Complex (Compound 6B) Packed in Column

2.5 g (0.06 mmol in terms of monomer complex) of compound 6B obtained in Example 6 was suspended in methanol, and poured into a column through a packer. Then, methanol was fed with a HPLC pump. When the pressure became constant, the packing was considered to be completed. Next, this column was placed in a column oven at 60°C, and a mixture solution of 6 ml (51.4 mmol) of acetophenone, 25 ml of a formic acid-triethylamine (5:2 (mole ratio)) azeotrope, and 125 ml of methanol was continuously fed at a flow rate of 0.05 ml/min by using an HPLC pump. The amount of moles of formic acid was approximately 3.6 times that of acetophenone.

After 2 hours from the start of the reaction, the conversion was 100%, and the selectivity was 97% ee. The feeding of the liquid was continued for 10 hours under the same conditions, and the reaction liquid having passed through the column was analyzed by GC every one hour. The results of each analysis were that the conversion was 100% and the selectivity was 97% ee.

## Claims

1. A solid-supported ruthenium complex represented by general formula (1): 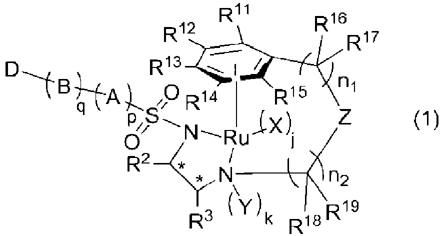 wherein
each * represents an asymmetric carbon atom;
R² and R³ each independently represent an alkyl group having 1 to 10 carbon atoms; a phenyl group optionally having a substituent(s) selected from alkyl groups having 1 to 10 carbon atoms, alkoxy groups having 1 to 10 carbon atoms, and halogen atoms; or a cycloalkyl group having 3 to 8 carbon atoms and optionally having a substituent(s) selected from alkyl groups having 1 to 10 carbon atoms, provided that R² and R³, taken together, may form a ring;
R¹¹, R¹², R¹³, R¹⁴, and R¹⁵ each independently represent a hydrogen atom, an alkyl group having 1 to 10 carbon atoms, or an alkoxy group having 1 to 10 carbon atoms;
R¹⁶, R¹⁷, R¹⁸, and R¹⁹ each independently represent a hydrogen atom, a hydroxyl group, an alkyl group having 1 to 10 carbon atoms, or an alkoxy group having 1 to 10 carbon atoms, provided that R¹⁶ and R¹⁷, together with the carbon atom to which R¹⁶ and R¹⁷ are bonded, may form a carbonyl group, and provided that R¹⁸ and R¹⁹, together with the carbon atom to which R¹⁸ and R¹⁹ are bonded, may form a carbonyl group;
Z represents an oxygen atom or methylene (-CH₂-);
n₁ represents 1 or 2, and n₂ represents an integer of 1 to 3;
Y represents a hydrogen atom;
X represents a trifluoromethanesulfonyloxy group, a p-toluenesulfonyloxy group, a methanesulfonyloxy group, a benzenesulfonyloxy group, a hydrogen atom, or a halogen atom;
j and k each independently represent 0 or 1, provided that j+k is not 1;
A represents a divalent group selected from optionally substituted linear or branched alkylene groups having 1 to 20 carbon atoms, optionally substituted cycloalkylene groups having 3 to 20 carbon atoms, optionally substituted arylene groups having 6 to 20 carbon atoms, optionally substituted heterocyclic groups, an oxygen atom, a sulfur atom, 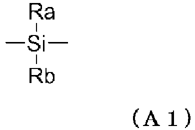 wherein Ra and Rb each independently represent a hydrogen atom, an alkyl group having 1 to 10 carbon atoms, or an aryl group having 6 to 10 carbon atoms, and 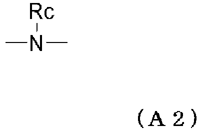 wherein Rc represents a hydrogen atom, an alkyl group having 1 to 10 carbon atoms, or an aryl group having 6 to 10 carbon atoms,
or a group formed by bonding two or more of these divalent groups, provided that either side of each of formula (A1) and formula (A2) may be bonded to B;
B represents 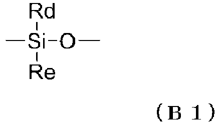 wherein Rd and Re each independently represent a hydrogen atom, an alkyl group having 1 to 10 carbon atoms, an aryl group having 6 to 10 carbon atoms, or an alkoxy group having 1 to 10 carbon atoms, 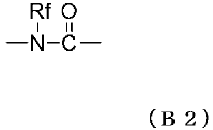 wherein Rf represents a hydrogen atom or an alkyl group having 1 to 10 carbon atoms, 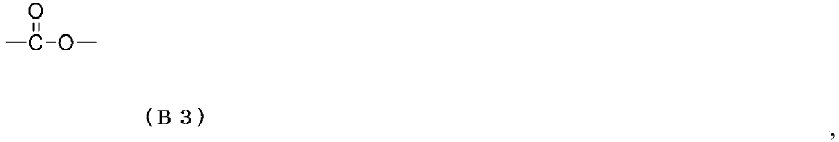 an oxygen atom, or a sulfur atom, provided that either side of each of formula (B1), formula (B2), and formula (B3) may be bonded to D;
D represents a solid support; and
p and q each independently represent 0 or 1.

2. A solid-supported ruthenium complex represented by general formula (2): 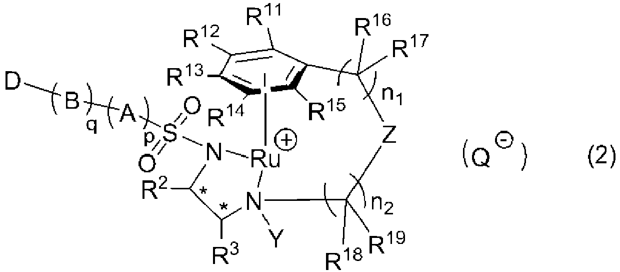 wherein
each * represents an asymmetric carbon atom;
R², R³, R¹¹, R¹², R¹³, R¹⁴, R¹⁵, R¹⁶, R¹⁷, R¹⁸, R¹⁹, Z, n₁, n₂, Y, A, B, D, p, and q are as defined in claim 1; and
Q⁻ represents a counter anion.

3. A solid-supported ruthenium complex represented by general formula (3): 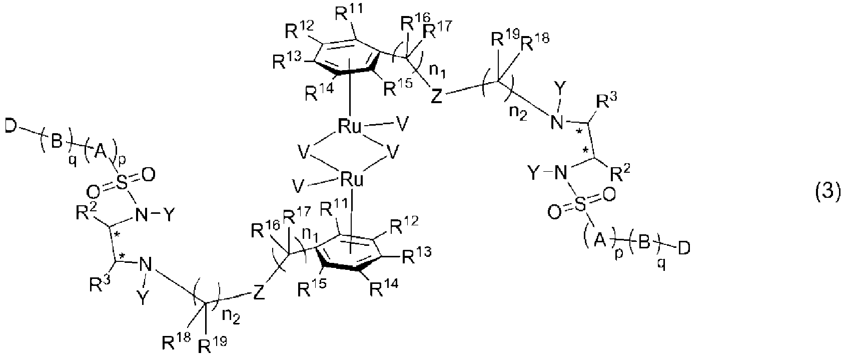 wherein
each * represents an asymmetric carbon atom;
R², R³, R¹¹, R¹², R¹³, R¹⁴, R¹⁵, R¹⁶, R¹⁷, R¹⁸, R¹⁹, Z, n₁, n₂, Y, A, B, D, p, and q are as defined in claim 1; and
each V represents a halogen atom.

4. A method for producing a reduction product, comprising
reducing an organic compound in the presence of the solid-supported ruthenium complex according to any one of claims 1 to 3 and a hydrogen donor.

5. A method for producing an optically active alcohol, comprising
reducing a carbonyl group of a carbonyl compound in the presence of the solid-supported ruthenium complex according to any one of claims 1 to 3 and a hydrogen donor.

6. A method for producing an optically active amine, comprising
reducing an imino group of an imine compound in the presence of the solid-supported ruthenium complex according to any one of claims 1 to 3 and a hydrogen donor.

7. The production method according to any one of claims 4 to 6, wherein the hydrogen donor is selected from formic acid, alkali metal formates, and alcohols having a hydrogen atom at an α-position carbon atom of a hydroxyl-bearing carbon.

8. The production method according to any one of claims 4 to 6, wherein the hydrogen donor is hydrogen.

9. A reduction catalyst, comprising the solid-supported ruthenium complex according to any one of claims 1 to 3.

10. The catalyst according to claim 9, wherein the reduction catalyst is an asymmetric reduction catalyst.
